# EUROPÄISCHE PATENTSCHRIFT

(11) **EP 0 564 984 B1**
(45) Veröffentlichungstag und Bekanntmachung des Hinweises auf die Patenterteilung: **20.08.1997**
(21) Anmeldenummer: 93105432.4
(22) Anmeldetag: 01.04.1993
(51) Int. Cl.: C07C 249/12, C07C 251/60, C07C 259/18, C07C 259/14, C07D 333/24, C07C 255/61, C07C 255/17, C07C 317/24

(54) **O-Iminooxymethylbenzoesäuren, Verfahren zu ihrer Herstellung und ihre Verwendung als Zwischenprodukte**
O-iminooxymethylbenzoic acids, their preparation and use as intermediates
Acides O-iminooxyméthylbenzoiques, leurs préparation et utilisation comme produits intermédiaires

(30) Priorität: 04.04.1992 DE 4211350
(43) Veröffentlichungstag der Anmeldung: 13.10.1993
(73) Patentinhaber: BASF Aktiengesellschaft, 67063 Ludwigshafen (DE)
(72) Erfinder: Benoit, Remy, Dr., W-6700 Ludwigshafen (DE); Sauter, Hubert, Dr., W-6800 Mannheim 1 (DE)

(56) Entgegenhaltungen:
- EP-A- 0 234 045
- EP-A- 0 272 487
- EP-A- 0 370 629
- EP-A- 0 463 488
- EP-A- 0 472 300

## Beschreibung

Die vorliegende Erfindung betrifft ein neues Verfahren zur Herstellung einer O-Iminooxymethylbenzoesäure der allgemeinen Formel I 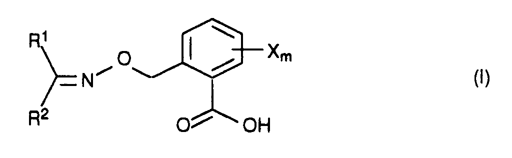 in der
- m: 0 oder eine ganze Zahl von 1 bis 3 bedeutet,
- X: ggf. verzweigtes C₁-C₄-Alkyl, ggf. verzweigtes C₁-C₄-Alkoxy, Nitro, Cyano, Halogen bedeutet,
- R¹,R²: gleich oder verschieden sind und Wasserstoff, Cyano, Hydroxy, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₆-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, Benzylamino, C₁-C₄-Alkylcarbonyl, ggf. subst. Phenylcarbonyl, ggf. subst. Benzylcarbonyl, C₁-C₄-Alkoxycarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Hetaryl-C₁-C₄-alkyl, ggf. subst. Hetaryl-C₂-C₄-alkenyl, ggf. subst. Hetaryloxy-C₁-C₄-alkyl, ggf. subst. Hetarylthio-C₁-C₄-alkyl, ggf. subst. Heterocyclyl, ggf. subst. Heterocyclyloxy bedeuten, oder
N(R⁶)₂ bedeuten, wobei die Bedeutungen von R⁶ gleich oder verschieden sind und Wasserstoff, C₁-C₆-Alkyl, ggf. subst. Phenyl bedeuten, oder
CO-N(R⁷)₂ bedeuten, wobei die Bedeutungen von R⁷ gleich oder verschieden sind und Wasserstoff, C₁-C₆-Alkyl, ggf. subst. Phenyl bedeuten,
wobei "ggf subst." neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoxyimino-C₁-C₂-alkyl, Aryl, Aryloxy, Benzyloxy, Hetaryl, Hetaryloxy, C₃-C₆-Cycloalkyl, Heterocyclyl, Heterocyclyloxy bedeuten, oder
- R¹ und R²: zusammen mit dem C-Atom, dessen Substituenten sie sind, einen carbo- oder heterocyclischen Ring bilden können, der durch die unter "ggf. susbt." oben genannten Reste substituiert sein kann, oder
- R¹ oder R²: Halogen bedeutet oder die Gruppe 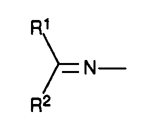 den Rest 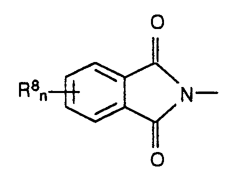 bedeutet, wobei
n die ganzen Zahlen von 1 bis 4 bedeutet, und
die Reste R⁸ gleich oder verschieden sind und Wasserstoff, Halogen, Cyano, Nitro, ggf. subst. C₁-C₄-Alkyl, C₁-C₄-Alkoxy, C₁-C₄-Halogenalkyl, C₁-C₄-Halogenalkoxy, Aryl, Aryloxy, Benzyloxy, Hetaryl, Hetaryloxy bedeuten,
indem man ein Oxim der allgemeinen Formel II 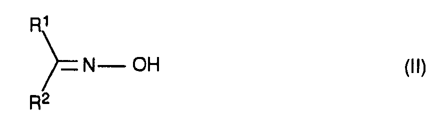 in dem R¹ und R² die oben genannte Bedeutung haben, mit einem Lacton der allgemeinen Formel III, 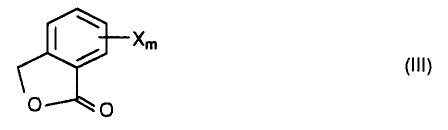 in dem X und m die oben gegebene Bedeutung haben, gegebenenfalls in Gegenwart einer Base oder eines Verdünnungsmittels oder ihrer Mischung umsetzt.

Die vorliegende Erfindung betrifft außerdem o-Iminooxymethylbenzoesäuren I sowie deren Verwendung als Zwischenprodukte. Desweiteren betrifft die Erfindung neue o-Iminooxymethylbenzoesäure-chloride IV und -cyanide VI.

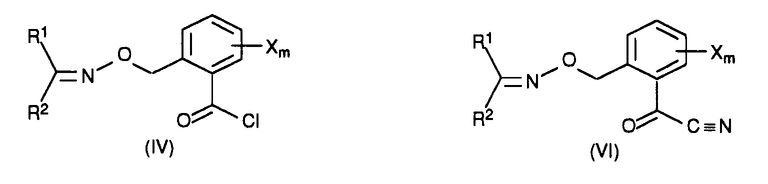

o-Iminooxymethylbenzoesäuren werden in EP 234045 (S. 97) beschrieben. Die Synthese geht dort aus vom Benzylbromid i, das seinerseits durch Bromierung nach an sich bekannten Methoden (siehe Organikum, S. 212) aus o-Methylbenzoesäuremethylester herstellbar ist und das anschließend mit dem Salz eines Oxims umgesetzt wird (EP 234045, S. 97). In der letzten Stufe wird die Estergruppe in üblicher Weise verseift um die Säure freizusetzen.

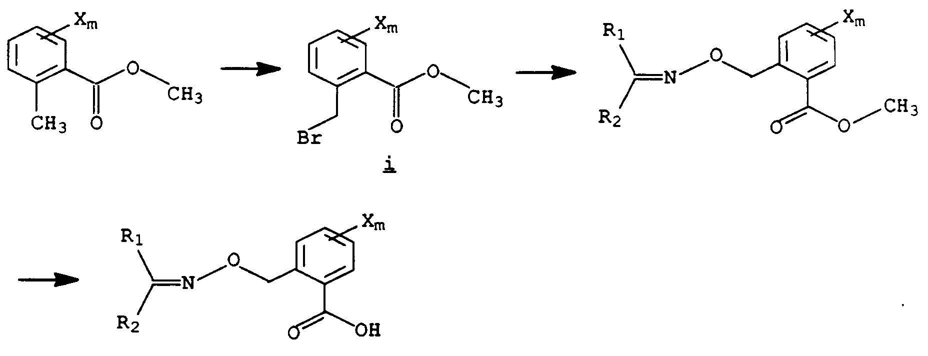

Die Herstellung und Umsetzung des Benzylbromids i stellt ein besonderes Problem dar. Das Arbeiten mit Brom oder N-Bromsuccinimid ist insbesondere im großen Maßstab aufwendig und umständlich. Weiterhin muß man bromhaltige Abfallprodukte entsorgen.

Diese Herstellmethode hat den entscheidenden Nachteil, ein langes und mehrstufiges Verfahren zu sein. Die Ausbeute und noch viel mehr die Raum-Zeit-Ausbeute sind schlecht. Infolgedessen ist die Herstellung der Zwischenprodukte der Formel I nach dem bekannten Verfahren sehr umständlich, wobei unerwünschte Abfallprodukte anfallen.

Der Erfindung lag daher die Aufgabe zugrunde, die Verbindungen I auf einem technisch besser gangbaren Weg herzustellen. Diese Aufgabe wird durch das oben beschriebene Verfahren gelöst.

Die Erfindung betrifft ferner ein Verfahren zur Herstellung von o-Iminooxymethylbenzoesäure I, welches dadurch gekennzeichnet ist, daß man
a) ein Oxim der allgemeinen Formel II 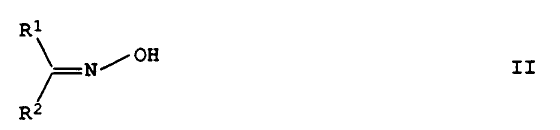 in Gegenwart eines Verdünnungsmittels mittels einer Base in das entsprechende Salz überführt,
b) dieses mit einem Lacton der allgemeinen Formel III 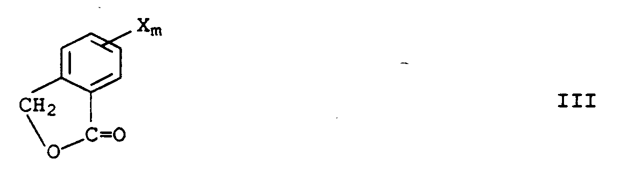 mischt und
c) das Gemisch in Lösung in einem Temperaturbereich zwischen 20 und 250°C weiter umsetzt, oder
d) das Verdünnungsmittel abdestilliert und das Gemisch in einem Temperaturbereich zwischen 50 und 250°C in der Schmelze umsetzt.

Als Basen eignen sich insbesondere Alkalimetallhydroxide wie Natrium- und Kaliumhydroxid sowie Alkalimetall- und Erdalkalimetallalkoholate wie Natriummethanolat, Natriumethanolat und Kaliummethanolat sowie Metallhydride wie Natriumhydrid, die Alkalimetallalkoholate und die Metallhydride werden besonders bevorzugt.

In der Regel führt man den Verfahrensschritt (a) in Gegenwart eines Lösungs- oder Verdünnungsmittels durch, wobei die Reaktionstemperatur normalerweise zwischen 0 und 100°C, bevorzugt zwischen 20 und 80°C liegt.

Als Lösungs- oder Verdünnungsmittel kommen aromatische Kohlenwasserstoffe wie Benzol, Toluol und o-, m-, p-Xylol, Alkohole wie Methanol, Ethanol und Isopropanol, Ether wie Dimethoxyethan, Tetrahydrofuran oder Flüssigkeiten wie Acetonitril, Dimethylformamid, N-Methylpyrrolidon, Dimethylpropylenharnstoff oder Gemische der genannten Lösungsmittel in Betracht. Besonders bevorzugt werden Methanol und Ethanol, Dimethylformamid und N-Methylpyrrolidon.

Die Menge an Base ist nicht kritisch. Für eine vollständige Überführung des Oxims II in dem entsprechenden Salz (Oximat) werden mindestens äquimolare Mengen an Base benötigt; bevorzugt verwendet man einen Überschuß von 1 bis 6 mol-% an Base, bezogen auf die Menge an Lacton III.

Die Salze des Oxims (Oximat) II werden vorteilhaft ohne Isolierung aus der Reaktionsmischung mit den Lactonen der Formel III gemischt und bei gleichzeitiger Abtrennung des Lösungsmittels zusammengeschmolzen oder in dem Verdünnungsmittel weiter umgesetzt.

Im allgemeinen führt man den Verfahrensschritt (d) bei einer Temperatur zwischen 50 und 250°C, vorzugsweise zwischen 160 und 200°C durch. Hierbei geht mit abnehmender Verdünnungsmittelmenge das Lacton III vom gelösten in den flüssigen Zustand über und man erhält eine gut mischbare Lösung des Oximats im Lacton III.

Der Verfahrensschritt (c) wird bei einer Temperatur zwischen 50 und 200°C, vorzugsweise zwischen 60 und 160°C, durchgeführt.

Normalerweise setzt man Oximat und Lacton in etwa stöchiometrischem Verhältnis ein, jedoch kann in manchen Fällen auch ein Überschuß der einen oder anderen Komponente, etwa bis zu 10 mol-%, empfehlenswert sein.

Bei den Verfahrensstufen (a) bis (d) sind keine besonderen Bedingungen bezüglich des Druckes erforderlich; zweckmäßigerweise arbeitet man daher bei Normaldruck.

Nach Beendigung der Reaktion verdünnt man das Reaktionsgemisch oder die Schmelze mit Wasser. Die erhaltene Lösung wird zur Freisetzung der o-Iminooxymethylbenzoesäure I angesäuert, bevorzugt mit einer anorganischen Säure wie Salzsäure oder Schwefelsäure. Die weitere Aufarbeitung erfolgt wie üblich.

Die nach dem erfindungsgemäßen Verfahren hergestellten Verbindungen der Formel I können grundsätzlich als E/Z-Gemische bezüglich der C=N-Doppelbindung anfallen, sofern R¹ und R² verschieden sind. In der Regel erhält man jedoch weit überwiegend, wenn nicht ausschließlich, nur ein Isomeres, wenn R¹ und R² sterisch genügend unterschiedlich sind.

Das erfindungsgemäße Verfahren kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Die beschriebene Herstellmethode läßt sich mit Erfolg zur Synthese der definitionsgemäßen o-Iminooxymethylbenzoesäuren der Formel I anwenden, vor allem auf solche Verbindungen, in denen die Substituenten R₁, R₂, X und m die folgende Bedeutung haben:
m 0 oder eine ganze Zahl von 1 bis 2 (insbesondere 0 oder 1) bedeutet.
X Methyl, Ethyl, Isopropyl, Methoxy, Nitro, Cyano, Fluor, Chlor, Brom bedeutet.
R₁ Wasserstoff; Cyano; C₁-C₆-Alkyl (insbesondere Methyl, Ethyl n-Propyl, iso-Propyl, Butyl); C₃-C₆-Cycloalkyl (insbesondere Cyclopropyl); C₁-C₄-Alkylthio (insbesondere Methylthio, Ethylthio, n-Propylthio, iso-Propylthio), C₁-C₄-Alkylthioalkyl (insbesondere Methylthiomethylen); Arylthioalkyl (insbesondere Phenylthiomethylen); C₁-C₄-Alkoxy (insbesondere Methoxy, Ethoxy, n-Propyloxy, Iso-Propyloxy); C₁-C₄-Alkoxyalkyl (insbesondere Methoxymethylen, Ethoxymethylen); Aryloxyalkyl (insbesondere Phenoxymethylen); C₁-C₄-Alkylamino (insbesondere Methylamino, Ethylamino, n-Propylamino, iso-Propylamino); C₁-C₃-Dialkylamino (insbesondere Dimethylamino, Diethylamino); Benzylamino; Benzylthio; Benzyloxy; Benzyl; Vinyl; E-Chlorvinyl; E-Bromvinyl; -OH; NH₂; -CO-NHCH₃; -CONHEt; -CONHnPr; -CONHiPr; -CON(CH₃)₂; -CON(Et)₂; -COOCH₃; -COOEt; -COOnPr; -COOiPr bedeutet.
R₂ C₁-C₄-Alkyl (insbesondere Methyl, Ethyl, n-Propyl, i-Propyl, n-Butyl); Phenyl [ggf. substituiert mit 0 bis 3 Gruppen ausgewählt aus C₁-C₄-Alkoxy (insbesondere Methoxy, Ethoxy, i-Propoxy); C₁-C₄-Alkylthio (insbesondere Methylthio, Ethylthio); Fluor; Chlor; Brom; Iod; C₁-C₄-Alkyl (insbesondere Methyl, Ethyl, n-Propyl, iso-Propyl, n-Butyl, tert.-Butyl); C₃-C₆-Cycloalkyl (insbesondere Cyclopropyl, Cyclopentyl, Cyclohexyl); Nitro; Cyano; Trifluormethyl; Trichlormethyl; Phenyl; Phenoxy; Benzyl; Benzyloxy; Hetaryl (insbesondere Pyridinyl, Pyrimidinyl, Pyrazinyl, Pyridazinyl, Thienyl, Furyl, Pyrrolyl, Pyrazolyl, Imidazolyl, Thiazolyl, Isothiazolyl, Oxazolyl, Isoxazolyl); Heterocyclyl (insbesondere Morpholinyl, Pyrrolidinyl, Thiomorpholinyl, Piperidinyl, Piperazinyl, Tetrahydrofuryl, Tetrahydrothienyl, Tetrahydropyranyl, Tetrahydrothiopyranyl); Hetaryloxy (insbesondere Pyridinyloxy, Pyrimidinyloxy, Pyrazinyloxy, Pyridazinyloxy); C₁-C₄-Alkylcarbonyl (insbesondere -COMe,
   -COEt, - COnPr, -COiPr, -COnBu, -CO tert.-Bu); C₁-C₄-Alkoxycarbonyl (insbesondere -COOMe, -COOEt, -COOnPr, -COOiPr, -COOnBu, -COO tert.-Bu); C₁-C₄-Alkylaminocarbonyl (insbesondere -CONHMe, -CONHEt, -CONHPr, -CONHiPr, -CONHnBu, -CONH tert.-Bu); C₁-C₄-Dialkylaminocarbonyl (insbesondere -CON(Me)₂,
   -CON(Et)₂); C₁-C₄-Dialkylamino (insbesondere -N(Me)₂,
   -N(Et)₂); Ph(Me)N-; Ph(Et)N-; -SO₂Me; -SO₂Et; -SOMe; -SOEt; -SO₂N(Me)₂; -SO₂N(Et)₂; -C(Me)=N-OMe; -C(Me)=N-OEt; -C(Me)=N-OnPr; -C(Me)=N-OiPr; -C(Me)=N-OnBu; -C(Me)=N-OtertBu;
   -C(Me)=N-OBenzyl; -C(Et)=N-OMe; -C(Et)=N-OEt; -C(Et)=N-OnPr; -C(Et)=N-OiPr; -C(Et)=N-OnBu; -C(Et)=N-OtertBu; -C(Et)=N-OBenzyl; -C(nPr)=N-OMe; -C(nPr)=N-OEt; -C(nPr)=N-OnPr; -C(NPr)=N-OiPr; -C(nPr)=N-OnBu; -C(nPr)=N-OtertBu; -C(nPr)=N-OBenzyl; -C(iPr)=N-OMe; -C(iPr)=N-OEt; -C(iPr)=OnPr; -C(iPr)=N-OiPr; -C(iPr)=N-OnBu; -C(iPr)=N-OtertBu; -C(iPr)=N-OBenzyl];
Naphthyl [ggf. substituiert mit 0 bis 3 Gruppe ausgewählt aus C₁-C₄-alkyl (insbesondere Methyl, Ethyl, n-Propyl, iso-Propyl); C₁-C₄-Alkoxy (insbesondere Methoxy, Ethoxy, n-Propyloxy, iso-Propyloxy); C₁-C₄-Alkylthio (insbesondere Methylthio, Ethylthio); F, Cl, Br, NO₂, CN];
Hetaryl (insbesondere Pyridinyl, Pyrimidinyl, Pyrazinyl, Pyridazinyl, Triazinyl, Chinolyl, Chinoxalyl, Naphthyridinyl, Trizolyl, Imidazolyl, Pyrazolyl, Thiazolyl, Isothiazolyl, Oxazolyl, Isoxazolyl, Oxadiazolyl, Thiadiazolyl, Thienyl, Furyl, Pyrrolyl, Benzothienyl, Benzofuryl, Indolyl, Benzimidazolyl, Benzopyrazolyl, Benzothiazolyl, Benzoisothiazolyl, Benzoxazolyl, Benzoisoxazolyl) wobei das Hetaryl durch 0 bis 2 Substiuenten, ausgewählt aus Methyl, Ethyl, i-Propyl, n-Propyl, n-Butyl, tert.-Butyl, Cyclopropyl, F, Cl, Br, I, Methoxy, Ethoxy, n-Propoxy, i-Propoxy, Cyano, Nitro, COOMe, COOEt, COOnPr, COOiPr, CONHMe, CONHEt, CON(Me)₂, CON(Et)₂, Methylthio, Ethylthio, -COCH₃, -COEt, -COiPr,
   -COnPr, Phenyl und Phenoxy substituiert sein kann;
Phenylcarbonyl [wobei der Phenylring durch 0 bis 3 Substituenten, ausgewählt aus: Methyl, Ethyl, n-Propyl, i-Propyl, n-Butyl, tert.-Butyl, Cyclopropyl, MeO, EtO, nPrO, iPrO, MeS, EtS, F, Cl, Br, I, NO₂, Cyano, Trifluormethyl substituiert sein kann]
bedeutet oder der Rest 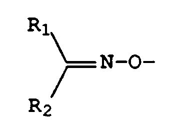 die folgende Bedeutung haben kann:

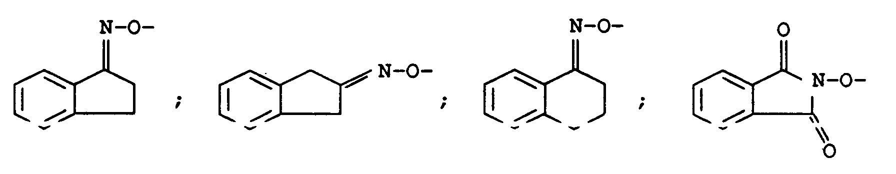

Die o-Iminooxymethylbenzoesäuren der Formel I werden überraschenderweise nach dem erfindungsgemäßen Verfahren in hoher Ausbeute und ausgezeichneter Reinheit erhalten. Im Hinblick auf den Stand der Technik war dies nicht zu erwarten. Insbesondere war es keinesfalls vorauszusehen, daß sich das Salz des Oxims (Oximat) in dieser Reaktion bei hohen Temperaturen (100-160°C) gut umsetzen lassen würde, ohne sich zu zersetzen. Das erfindungsgemäße Verfahren besitzt gegenüber dem Stand der Technik eine Reihe von Vorteilen. Es ist im technischen Maßstab in sehr einfacher Weise durchführbar. Es benötigt nicht die Verwendung von Brom oder evtl. Chlor oder N-Bromsuccinimid oder N-Chlorsuccinimid. Es vermeidet damit die Entstehung halogenhaltiger Abfall-Produkte und ist deswegen technisch einfacher und problemloser. Zusätzlich werden durch Halogen bedingte Korrosionsprobleme vermieden. Ein wesentlicher Vorteil dieses Verfahrens ist weiterhin, daß es in einen einstufigen technisch einfachen und problemlosen Verfahren zu den Säuren der Formel I führt.

Von Vorteil ist es ferner, daß bei einer Variante des Verfahrens das intermediär erzeugte Alkali-Oximat nicht als Feststoff isoliert werden muß, sondern daß ein fließender Übergang von einer Lösung bzw. Suspension des Oximats aus dem Verdünnungsmittel in die Schmelze (Oximat + Lacton III) erfolgt. Dies wird durch Zugabe des Lactons III zu dem Salz des Oxims II in Gegenwart eines Verdünnungsmittels und anschließende Abdestillation des Verdünnungsmittels erreicht.

Bei einer anderen Variante kann man das Oximat mit dem Phthalid im Lösungsmittel weiter umsetzen. Die Flexibilität der Reaktionsführung im angegebenen Rahmen ermöglicht es, die für den jeweiligen Einzelfall optimalen Bedingungen zu wählen.

Die o-Iminooxymethylbenzoesäuren I sind wertvolle Zwischenprodukte für die Herstellung von o-Iminooxymethylphenylestern IX und X, die im Pflanzenschutz insbesondere als Fungizide Verwendung finden (vgl. EP-A 370629; EP-A 414 155; EP-A 426 460; EP-A 460 575; WO 70/07493).

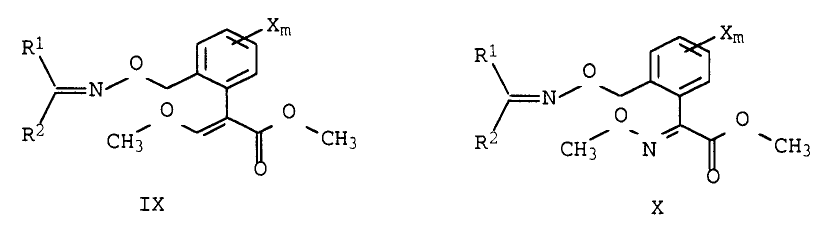

Im folgenden Reaktionsschema werden zwei besonders bevorzugte Synthesewege zur Herstellung der Verbindungen IX aus o-Iminooxymethylbenzoesäure dargestellt:

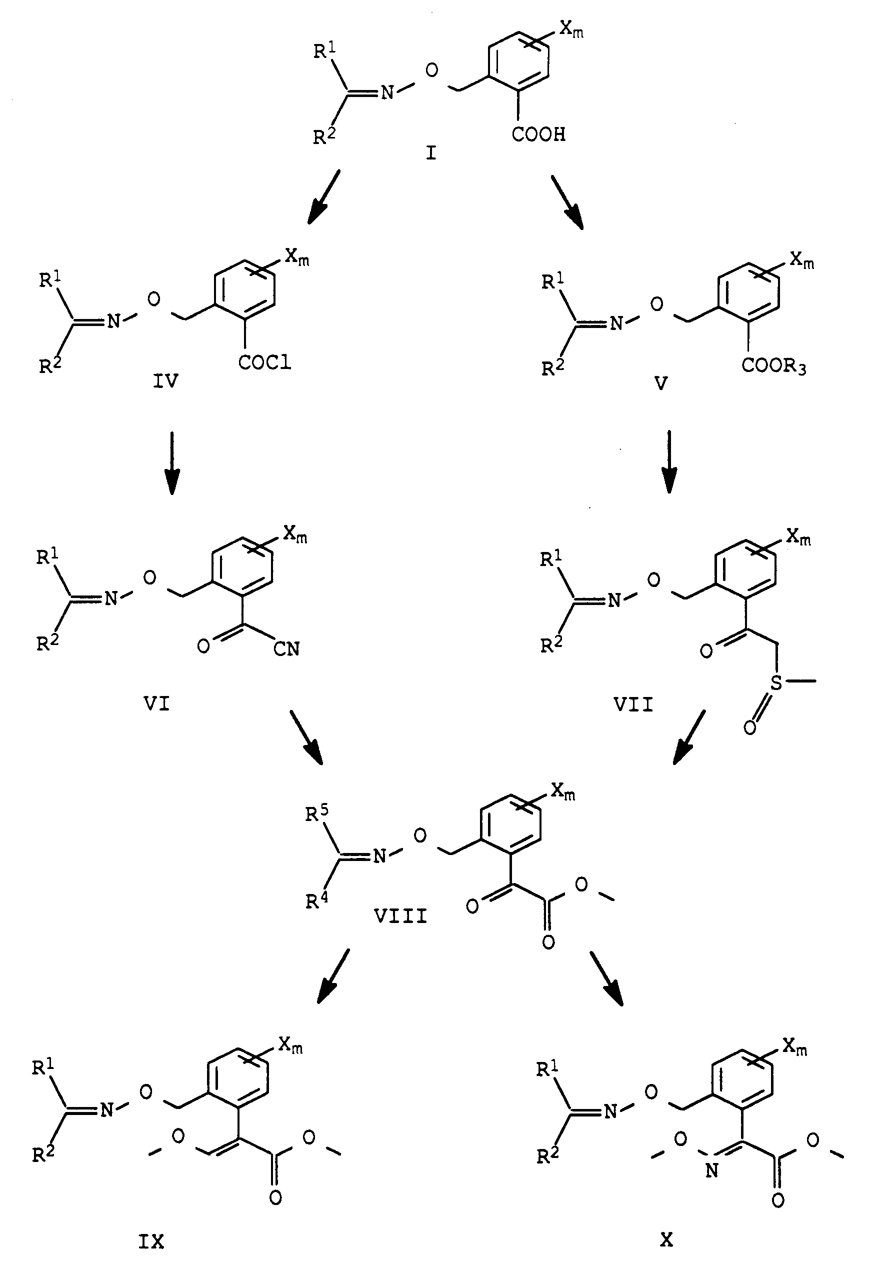

Nach diesen Verfahren überführt man vorteilhaft die Benzoesäuren I in ihre Säurechloride IV (vgl. Organikum, VEB Deutscher Verlag der Wissenschaften, 16. Auflage, Berlin 1986, Seite 423 f.) und stellt aus diesen, unter Verwendung eines wässrig organischen Zweiphasensystems und in Gegenwart eines Phasentransferkatalysators, die entsprechenden Benzoylcyanide VI her [Tetrahedron Letters, 2275 (1974)].

Die Benzoylcyanide IV werden anschließend mit niederen Alkoholen nach der Pinner-Reaktion umgesetzt [vgl. Angew. Chemie 94, 1 (1982)], wobei Phenylglyoxylsäureester VIII entstehen. Anschließend setzt man die α-Ketoester VIII entweder mit o-Methylhydroxylamin (oder einem Salz davon) um, um die bis-Oximether des Typs X zu erreichen, oder man setzt sie mit einem Methoxymethylenierungsreagenz wie Methoxymethylentriphenylphosphoran (vgl. EP 0044 448, 04.07.1980) um, um die Acrylester IX herzustellen. Bei einem zweiten bekannten Verfahren überführt man zuerst die Säure I in ihren entsprechenden Ester V (vgl. Organikum Seite 499). Diese Ester V werden dann in Gegenwart von Base und Dimethylsulfoxid in das β-Ketosulfoxid VII umgewandelt (J. Am. Chem. Soc. 88, 5498 (1966)). Anschließend werden die α-Ketoester VIII durch Bromierung der Verbindung VII in Gegenwart einer Base und Umlagerung in eine Säure nach der Pummerer-Reaktion gewonnen (vgl. J. Am Chem. Soc. 88, 5498 (1966) und Synthesis, 41 (1982)).

Für die Zwischenprodukte I, IV, V, VI und VII haben die Reste R₁, R₂, X und m bevorzugt die folgende Bedeutung:
m 0 oder eine ganze Zahl von 1 bis 2 (insbesondere 0 oder 1)
X bedeutet Methyl, Ethyl, Isopropyl, Methoxy, Nitro, Cyano, Fluor, Chlor oder Brom.
R₁ steht für Wasserstoff; Cyano; C₁-C₆-Alkyl (insbesondere Methyl, Ethyl n-Propyl, iso-Propyl, Butyl); C₃-C₆-Cycloalkyl (insbesondere Cyclopropyl); C₁-C₄-Alkylthio (insbesondere Methylthio, Ethylthio, n-Propylthio, iso-Propylthio), C₁-C₄-Alkylthioalkyl (insbesondere Methylthiomethylen); Arylthioalkyl (insbesondere Phenylthiomethylen); C₁-C₄-Alkoxy (insbesondere Methoxy, Ethoxy, n-Propyloxy, Iso-Propyloxy); C₁-C₄-Alkoxyalkyl (insbesondere Methoxymethylen, Ethoxymethylen); Aryloxyalkyl (insbesondere Phenoxymethylen); C₁-C₄-Alkylamino (insbesondere Methylamino, Ethylamino, n-Propylamino, iso-Propylamino); C₁-C₃-Dialkylamino (insbesondere Dimethylamino, Diethylamino); Benzylamino; Benzylthio; Benzyloxy; Benzyl; Vinyl; E-Chlorvinyl; E-Bromvinyl; -OH; NH₂; -CO-NHCH₃;
   -CONHEt; -CONHnPr; -CONHiPr; -CON(CH₃)₂; -CON(Et)₂; -COOCH₃; -COOEt; -COOnPr; -COOiPr.
R₂ bedeutet C₁-C₄-Alkyl (insbesondere Methyl, Ethyl, n-Propyl, i-Propyl, n-Butyl); Phenyl [ggf. substituiert mit 0 bis 3 Gruppe ausgewählt aus C₁-C₄-Alkoxy (insbesondere Methoxy, Ethoxy, i-Propoxy); C₁-C₄-Alkylthio (insbesondere Methylthio, Ethylthio); Fluor; Chlor; Brom; Iod; C₁-C₄-Alkyl (insbesondere Methyl, Ethyl, n-Propyl, iso-Propyl, n-Butyl, tert.-Butyl); C₃-C₆-Cycloalkyl (insbesondere Cyclopropyl, Cyclopentyl, Cyclohexyl); Nitro; Cyano; Trifluormethyl; Trichlormethyl; Phenyl; Phenoxy; Benzyl; Benzyloxy; Hetaryl (insbesondere Pyridinyl, Pyrimidinyl, Pyrazinyl, Pyridazinyl, Thienyl, Furyl, Pyrrolyl, Pyrazolyl, Imidazolyl, Thiazolyl, Isothiazolyl, Oxazolyl, Isoxazolyl); Heterocyclyl (insbesondere Morpholinyl, Pyrrolidinyl, Thiomorpholinyl, Piperidinyl, Piperazinyl, Tetrahydrofuryl, Tetrahydrothienyl, Tetrahydropyranyl, Tetrahydrothiopyranyl); Hetaryloxy (insbesondere Pyridinyloxy, Pyrimidinyloxy, Pyrazinyloxy, Pyridazinyloxy); C₁-C₄-Alkylcarbonyl (insbesondere -COMe, -COEt, - COnPr, -COiPr, -COnBu, -CO tert.-Bu); C₁-C₄-Alkoxycarbonyl (insbesondere -COOMe, -COOEt, COOnPr, COOiPr, COOnBu, COO tert.-Bu); C₁-C₄-Alkylaminocarbonyl (insbesondere -CONHMe, CONHEt, -CONHPr, -CONHiPr, -CONHnBu, -CONH tert.-Bu); C₁-C₄-Dialkylaminocarbonyl (insbesondere -CON(Me)₂, -CON(Et)₂); C₁-C₄-Dialkylamino (insbesondere -N(Me)₂, -N(Et)₂);
   Ph(Me)N-; Ph(Et)N-; -SO₂Me; -SO₂Et; -SOMe; -SOEt; -SO₂N(Me)₂; -SO₂N(Et)₂; -C(Me)=N-OMe; -C(Me)=N-OEt; -C(Me)=N-OnPr; -C(Me)=N-OiPr; -C(Me)=N-OnBu; -C (Me)=N-OtertBu; -C(Me)=N-OBenzyl; -C(Et)=N-OMe; -C(Et)=N-OEt; -C(Et)=N-OnPr; -C(Et)=N-OiPr; -C(Et)=N-OnBu;
   -C(Et)=N-OtertBu;-C(Et)=N-OBenzyl; -C(nPr)=N-OMe; -C(npr)=N-OEt;
   -C(nPr)=N-OnPr; -C(NPr)=N-OiPr; -C(nPr)=N-OnBu; -C(nPr)=N-OtertBu; -C(nPr)=N-OBenzyl; -C(iPr)=N-OMe;
Naphthyl [ggf. substituiert mit 0 bis 3 Gruppe ausgewählt aus C₁-C₄-alkyl (insbesondere Methyl, Ethyl, n-Propyl, iso-Propyl); C₁-C₄-Alkoxy (insbesondere Methoxy, Ethoxy, n-Propyloxy, iso-Propyloxy); C₁-C₄-Alkylthio (insbesondere Methylthio, Ethylthio); F, Cl, Br, NO₂, CN];
Hetaryl (insbesondere Pyridinyl, Pyrimidinyl, Pyrazinyl, Pyridazinyl, Triazinyl, Chinolyl, Chinoxalyl, Naphthyridinyl, Trizolyl, Imidazolyl, Pyrazolyl, Thiazolyl, Isothiazolyl, Oxazolyl, Isoxazolyl, Oxadiazolyl, Thiadiazolyl, Thienyl, Furyl, Pyrrolyl, Benzothienyl, Benzofuryl, Indolyl, Benzimidazolyl, Benzpyrazolyl, Benzthiazolyl, Benzoisothiazolyl, Benzoxazolyl, Benzoisoxazolyl) wobei das Heteroaromat durch 0 bis 2 substiuent ausgewählt aus Methyl, Ethyl, i-Propyl, n-Propyl, n-Butyl, tert.-Butyl, Cyclopropyl, F, Cl, Br, I, Methoxy, Ethoxy, n-Propoxy, i-Propoxy, Cyano, Nitro, COOMe, COOEt, COOnPr, COOiPr, CONHMe, CONHEt, CON(Me)₂, CON(Et)₂, Methylthio, Ethylthio, -COCH₃, -COEt, -COiPr,
   -COnPr, Phenyl und Phenoxy substituiert sein kann; Phenylcarbonyl [wobei der Phenylring durch 0 bis 3 substituent ausgewählt aus: Methyl, Ethyl, n-Propyl, i-Propyl, n-Butyl, tert.-Butyl, Cyclopropyl, MeO, EtO, nPrO, iPrO, MeS, EtS, F, Cl, Br, I, NO₂, Cyano, Trifluormethyl substituiert sein kann]
oder der Rest 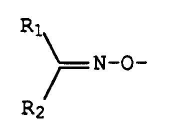 die folgende Bedeutung haben kann:

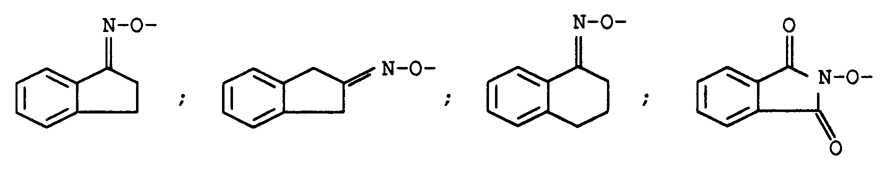

Für das Zwischenprodukt VIII haben R₄ und R₅ bevorzugt folgende Bedeutung:
R₄ bedeutet Benzyl; C₁-C₄-Alkylcarbonyl (z.B. -COMe, -COEt, -COnPr, -COiPr, -COnBu, -COtertBu); C₁-C₄-Alkoxycarbonyl (z.B. -COOMe, -COOEt, -COOnPr, -COOiPr, -COOnBu, -COOiBu, -COOtertBu); Phenyl; Naphthyl; Pyridinyl; Pyrimidinyl; Pyrazinyl; Pyridazinyl; Triazinyl; Chinolyl; Chinoxalyl; Naphthyridinyl; Triazolyl; Imidazolyl; Pyrrazolyl; Thiazolyl; Isothiazolyl; Oxazolyl; Isoxazolyl; Oxadiazolyl; Thiadiazolyl; Thienyl; Furyl; Pyrrolyl; Benzothienyl; Benzofuryl; Indolyl; Benzimidazolyl; Benzopyrazolyl; Benzothiazolyl; Benzoisothiazolyl; Benzoxazolyl; Benzoisoxazolyl.

Wobei jeder aromatische oder heteroaromatische Ring mit 1 bis 3 Gruppen ausgewählt aus: Wasserstoff; F; Cl; Br; I; Nitro; Cyano; C₁-C₄-Alkyl (z. B. Methyl, Ethyl, n-Propyl, i-Propyl, n-Butyl, i-Butyl, tert.-Butyl); C₁-C₄-Alkoxy (z.B. Methoxy, Ethoxy, n-Propoxy, i-Propoxy, n-Butoxy, i-Butoxy, tert.-Butoxy); Trifluormethyl; Trichlormethyl; Phenyl; Phenoxy; Benzyloxy substituiert sein kann.

R₅ bedeutet Wasserstoff; C₁-C₄-Alkyl (z.B. Methyl, Ethyl, n-Propyl, i-Propyl, n-Butyl, i-Butyl, tert.-Butyl); C₁-C₄-Alkylcarbonyl (z.B. -COMe, -COEt, -COnPr, -COiPr, -COnBu, -COiBu, -Cotert.-Bu); C₁-C₄-Alkoxycarbonyl (z.B. -COOMe, -COOEt, -COOnPr, -COOiPrn, -COOnBu, -COOiBu, -COO-tertBu); Cyano; C₁-C₄-Alkylthio (z. B. -SMe, -SEt, -SnPr, -SiPr, -SnBu, -SiBu, -StertBu); C₁-C₄-Alkoxy (z.B. -OMe, -OEt, -OnPr, OiPr, -OnBu, -OiBu, -OtertBu); C₃-C₆-Cycloalkyl (z.B. Cyclopropyl); NR₉R₁₀.

R₉ bedeutet C₁-C₄-Alkyl (z.B. Methyl, Ethyl, n-Propyl, i-Propyl); Phenyl C₁-C₄-Alkyl (z.B. Benzyl, 2-Phenylethyl, 1-Phenylethyl); Phenyl bedeutet.

R₁₀ bedeutet Wasserstoff; C₁-C₄-Alkyl (z.B. -Methyl, -Ethyl, n-Propyl, i-Propyl) bedeutet oder der Rest 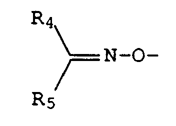 hat die folgende Bedeutung:

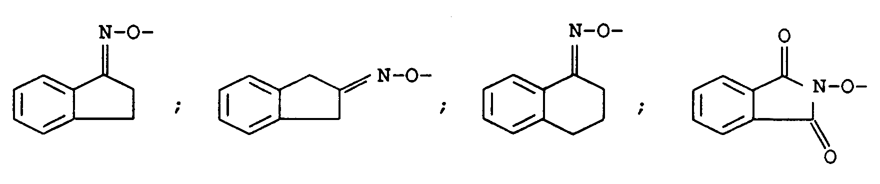

Die folgenden Beispiele erläutern das erfindungsgemäße Verfahren.

### Beispiel 1

### Verbindung 32 in Tabelle 1

25,4 g (0,15 mol) p-Chloracetophenonoxim werden mit 27 g einer 30 %igen (Gew.%) Natriummethylatlösung in 150 ml Methanol gelöst, 30 min unter Rückfluß erhitzt, das Methanol abdestilliert, 20 g (0,15 mol) Phthalid zugegeben und das Gemisch 2 Std. bei 180°C erhitzt. Die Heizung wird entfernt, bei 100°C 250 ml Wasser vorsichtig zugegeben, nach dem Abkühlen auf 20°C zweimal mit Methyl-tert.-butylether (MTBE) extrahiert, die wäßrige Phase mit konz. H₂SO₄ auf pH 2 angesäuert und der ausgefallene Feststoff abgesaugt. Man erhält 25,9 g (57 %) rohe Säure.

Warnung: In Form reiner Substanzen können sich die Natriumsalze von Acetophenonoximen oberhalb 150°C mit starker Energieentwicklung zersetzen. Deshalb empfehlen sich für die Umsetzung eher die folgenden Verfahrensvarianten (Beispiele 2 bis 5).

### Beispiel 2

### Verbindung 31 in Tabelle 1

7,5 g (0,05 mol) p-Methylacetophenonoxim werden unter Stickstoffgas mit 9 g einer 30 %igen Natriummethylatlösung in 30 ml CH₃OH gelöst, 1 Std. bei Rückfluß erhitzt, 6,7 g (0,05 mol) Phthalid zugegeben, das Methanol abdestilliert und das Gemisch 2 Std. bei 160°C erhitzt. Nach dem Abkühlen auf 20°C werden 100 ml Wasser zugegeben, zweimal mit MTBE extrahiert, und die wäßrige Phase mit konz. H₂SO₄ auf pH 2 angesäuert. Die dunkle ausgefallene Masse wird vom Wasser getrennt, in MTBE aufgelöst, getrocknet, eingeengt. Man erhält 9,6 g (68 %) Rohsäure.

### Beispiel 3

### Verbindung 31 in Tabelle 1

2,6 g NaH (55 %ig) werden vorgelegt, 7,5 g (0,05 mol) p-Methylacetophenonoxim in 50 ml Tetrahydrofuran (THF) gelöst, unter Stickstoffgas zugetropft, das Gemisch 1 Std. bei 60°C erhitzt, 50 ml THF zugegeben und auf 40°C gekühlt. Eine Lösung von 6,7 g (0,05 mol) Phthalid in 30 ml THF wird zugegeben, das Lösungsmittel abdestilliert und das Gemisch 2 Std. auf 160°C erhitzt. Nach dem Abkühlen werden 100 ml H₂O zugegeben, zweimal mit MTBE extrahiert, die wäßrige Phase mit konz. H₂SO₄ auf pH 2 angesäuert, die Suspension mit etwas Pentan verrührt, abgesaugt und der Niederschlag mit Wasser gewaschen. Nach dem Trocknen im Vakuumtrockenschrank erhält man 7,4 g (52 %) Rohsäure.

### Beispiel 4

### Verbindung 31 in Tabelle 1

2,6 g NaH (55 %ig) werden vorgelegt, 7,5 g (0,05 mol) p-Methylacetophenonoxim in 50 ml Dimethylformamid (DMF) unter N₂ zugetropft, das Gemisch 1 Std. bei 60°C weitergerührt, 50 ml DMF zugetropft und die Suspension 2 Std. bei Rückfluß erhitzt. Nach dem Abkühlen werden 200 ml H₂O zugegeben, mit konz. H₂SO₄ angesäuert, der Niederschlag abgesaugt, mit H₂O gewaschen und im Vakuumtrockenschrank getrocknet. Man erhält 9,3 g (66 %) Säure.

### Beispiel 5

### Verbindung 31 in Tabelle 1

2,6 g NaH (55 %ig) werden vorgelegt, 7,5 g (0,05 mol) p-Methylacetophenonoxim in 50 ml DMF unter N₂ zugetropft, das Gemisch 1 Std. bei 60°C weitergerührt, 50 ml DMF zugegeben, 6,7 g (0,05 mol) Phthalid in 50 ml DMF zugetropft, die Suspension 6 Std. bei 100°C und über Nacht bei 20°C gerührt. Danach wird Wasser (200 ml) zugegeben, mit konz. H₂SO₄ angesäuert, abgesaugt, der Niederschlag mit Wasser gewaschen und getrocknet. Man erhält 10,2 g (72 %) helle Kristalle der Säure. Fp = 152°C.

### Beispiel 6 (nicht erfindungsgemäß)

4 g (0,016 mol) der Säure (Verbindung Nr. 31, Tabelle 1) wird in 20 ml Methanol gelöst, 2,5 g (0,028 mol) SOCl₂ zugetropft und das Reaktionsgemisch 1 Std. unter Rückfluß erhitzt. Das Lösungsmittel wird am Rotationsverdampfer abgedampft, der Rückstand in MTBE aufgelöst, mit 10 %iger Na₂CO₃-Lösung gewaschen, mit Na₂SO₄ getrocknet und das Lösungsmittel verdampft. Man erhält 3,4 g (82 %) Öl.

### Beispiel 7 (nicht erfindungsgemäß)

12,5 g (0,041 mol) der Säure (Verbindung 32 in Tabelle 1) wird in 50 ml Methanol gelöst, 7,4 g (0,083 mol) SOCl₂ zugetropft und das Reaktionsgemisch 2 Std. unter Rückfluß gekocht. Das Lösungsmittel wird am Rotationsverdampfer abgedampft, der Rückstand in MTBE aufgelöst, dreimal mit einer 10 %igen wäßrigen Na₂CO₃-Lösung gewaschen, mit Na₂SO₄ getrocknet und das Lösungsmittel verdampft. Man erhält 11,2 g (86 %) Öl.

### Beispiel 8

### Verbindung 31 in Tabelle 1

45,8 g NaH (55 %ig) werden in 1000 ml DMF suspendiert, 149 g (1 Mol) p-Methylacetophenonoxim in 600 ml DMF unter N₂ zugetropft, das Gemisch 1 Std. bei 60°C weitergerührt, 200 ml DMF zugegeben, 134 g (1 mol) Phthalid in 500 ml DMF zugetropft, die Suspension 6 Std. bei 100°C und über Nacht bei 20°C gerührt. Danach wird Wasser (ca. 5 l) zugegeben, mit konz. H₂SO₄ angesäuert, abgesaugt, der Niederschlag mit Wasser gewaschen und getrocknet. Man erhält 262 g (92 %) helle Kristalle der Säure. Fp = 152°C.

### Beispiel 9

### Verbindung 46 in Tabelle 1

2,65 g NaH (55 %ig) werden in 50 ml DMF suspendiert, 7,05 g 3-Acetylthiophenoxim in 50 ml DMF unter N₂ zugetropft, das Gemisch 1 Std. bei 60°C weitergerührt, 6,7 g Phthalid in 50 ml DMF zugetropft, die Suspension 8 Std. bei 100°C gerührt. Nach Abkühlung wird ca. 150 ml H₂O zugegeben, mit konz. H₂SO₄ angesäuert, abgesaugt, der Niederschlag mit Wasser gewaschen und getrocknet. Man erhält 9 g (65 %) Kristalle der Säure. Fp = 149-151°C.

### Beispiel 10 (nicht erfindungsgemäß)

1,75 DMSO werden in 5 ml THF bei -40°C vorgelegt, 25 g Butyllithiumlösung (1,6 M) unter N₂ bei -40°C zugetropft, das Gemisch bis Ende der Entgasung bei 0°C weitergerührt, die Verbindung 31 Tab. 5 in 15 ml THF bei -40°C zugetropft und das Gemisch 2 Std. bei RT gerührt. Die Reaktionsmischung wird mit einer NH₄-Cl-Lösung hydrolisiert, mit 2 N HCl angesäuert, mit MTBE extrahiert, die organische Phase mit festem Na₂CO₃ neutralisiert, getrocknet und eingeengt. Nach chromatographischer Trennung erhält man 2,9 g (42 %) von der Verbindung 31 in der Tabelle 6.

### Beispiel 11 (nicht erfindungsgemäß)

2,9 g der Verbindung 31 in der Tabelle 6 werden in 30 ml Aceton gelöst, 1,3-Dibromdimethylhydantoin bei RT zugegeben, das Gemisch bei RT 15 min weitergerührt und eingeengt. Der Rückstand wird in 40 ml Methanol gelöst, 2 ml konz. HCl zugegeben und die Lösung 2 Std. bei RT weitergerührt. Die Reaktionsmischung wird in MRBE/Wasser aufgenommen, die organische Phase mit Na₂CO₃ und Wasser gewaschen, getrocknet und eingeengt- Das Produkt wird chromatographiert und man erhält 0,6 g eine Mischung aus p-Methylacetophenon und die Verbindung 31 in der Tabelle 4.
¹H·NMR (COCl₃/TMS): 2,25 (S,3H); 2,35 (S,3H); 3,90 (S,3H); 5,55 (S,2H); 7,05-7,80 (M,8H) ppm.

### Beispiel 12

### Verbindung 31 in der Tabelle 2

10 g der Verbindung 31 in der Tabelle 1 werden in 80 ml Toluol suspendiert, 8,3 g Pyridin und 2 Tropfen DMF zugegeben, bei 0°C 6,25 g SOCl₂ zugetropft, die Kühlung entfernt und das Gemisch 2 Std. weitergerührt. Der gelbe Niederschlag wird abgesaugt, mit Toluol gewaschen und die Mutterlauge eingeengt. Man erhält 5,3 g Rohprodukt.
¹H·NMR (COCl₃/TMS): 2,35 (S,6H); 5,70 (S,2H); 7,10-8,15 (M,8H) ppm.

### Beispiel 13 (nicht erfindungsgemäß)

### o-[1-(4-Methylphenyl)ethyliminoxymethyl]Phenylglyoxylsäureethylester Verbindung des Typs VIII mit R₄= 4-Methylphenyl; R₅= Methyl; R₁₁= Ethyl)

3,0 g der Verbindung 31 in der Tabelle 6 werden in 30 ml Aceton gelöst, 1,4-Dibromdimehylhydantoin bei RT zugegeben, das Gemisch bei RT 15 min weitergerührt und eingeengt. Der Rückstand wird in 40 ml Ethanol gelöst, 2 ml konz. HCl zugegeben und die Lösung 4 Std. bei RT weitergerührt. Die Reaktionsmischung wird in MTBE/Wasser aufgenommen, die organische Phase mit Na₂CO₃ neutralisiert, getrocknet und eingeengt. Das gebildete p-Methylacetophenon wird am Kugelrohr destilliert und der Sumpf chromatographiert. Man erhält 0,4 g o-[1-(4-Methylphenyl)ethyliminoxymethyl]phenylglyoxylsäureethylester.
¹H·NMR (COCl₃/TMS): 1,35 (t,34); 2,30 (S,3H); 4,40 (9,2H); 5,55 (S,2H); 7,05-7,80 (M,8H) ppm.
IR: 1733; 1683; 1195; 1038; 1012 cm-¹.

Nach den genannten Verfahren können beispielweise die folgenden o-Iminooxymethylbenzoesäuren der Formel I hergestellt werden.

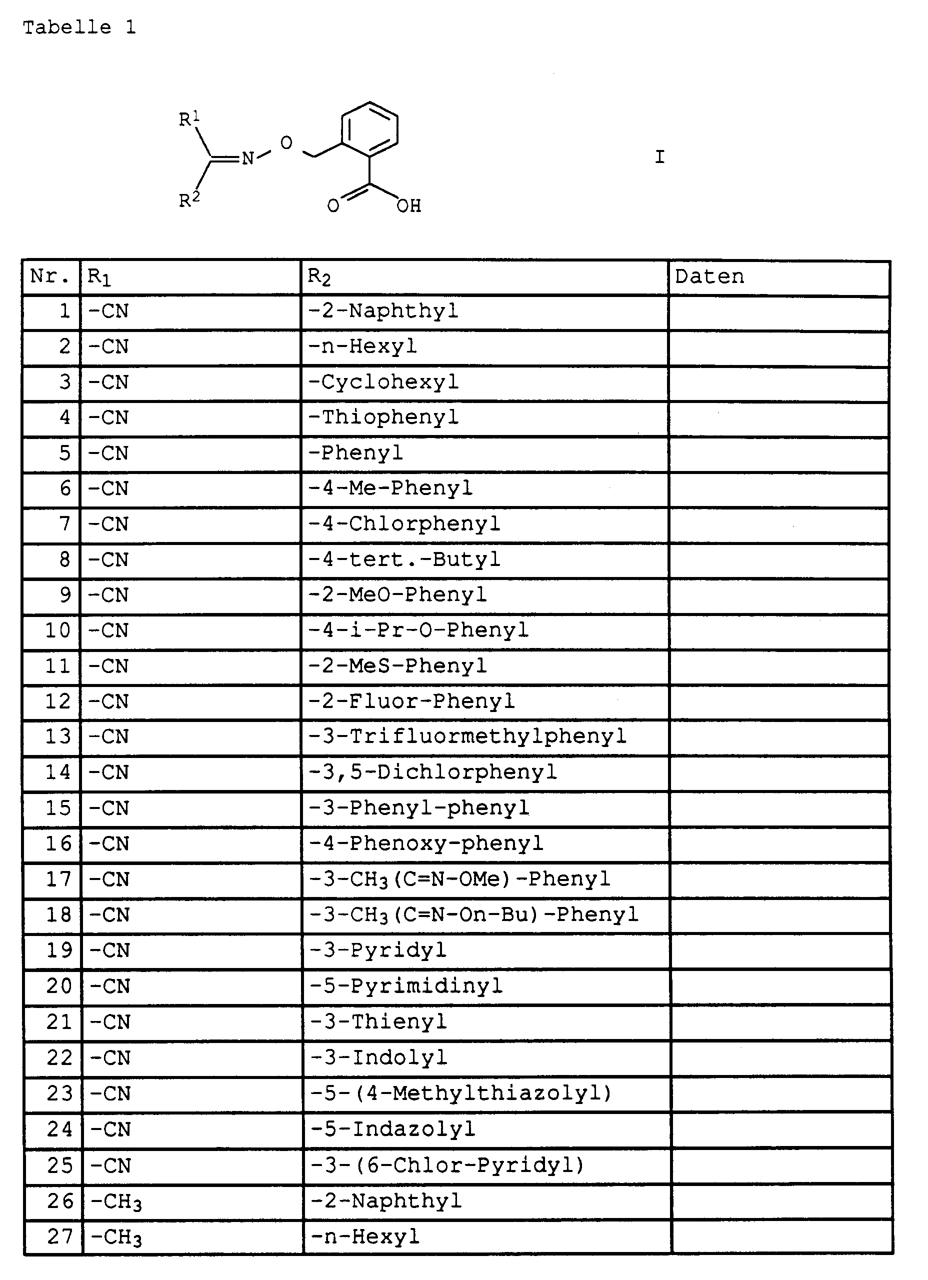

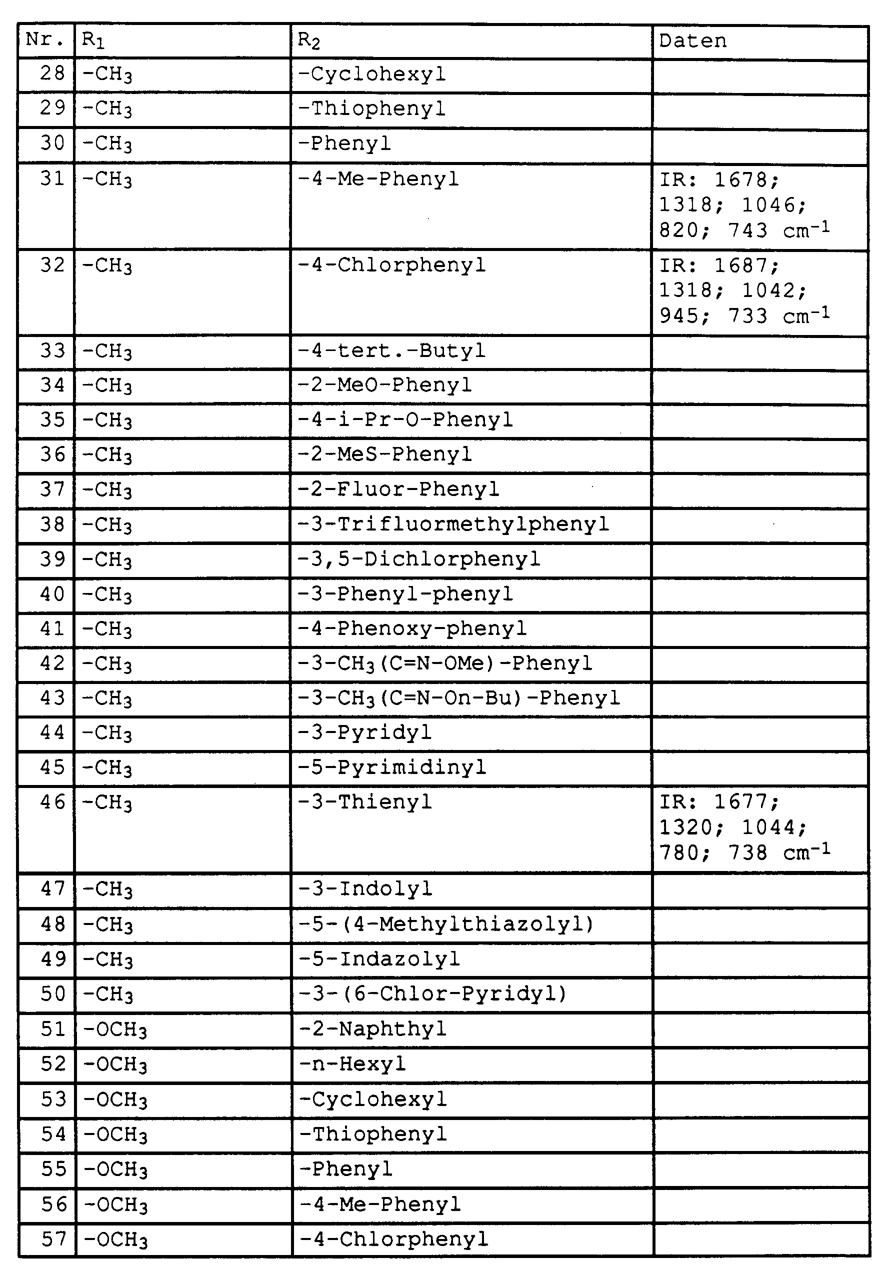

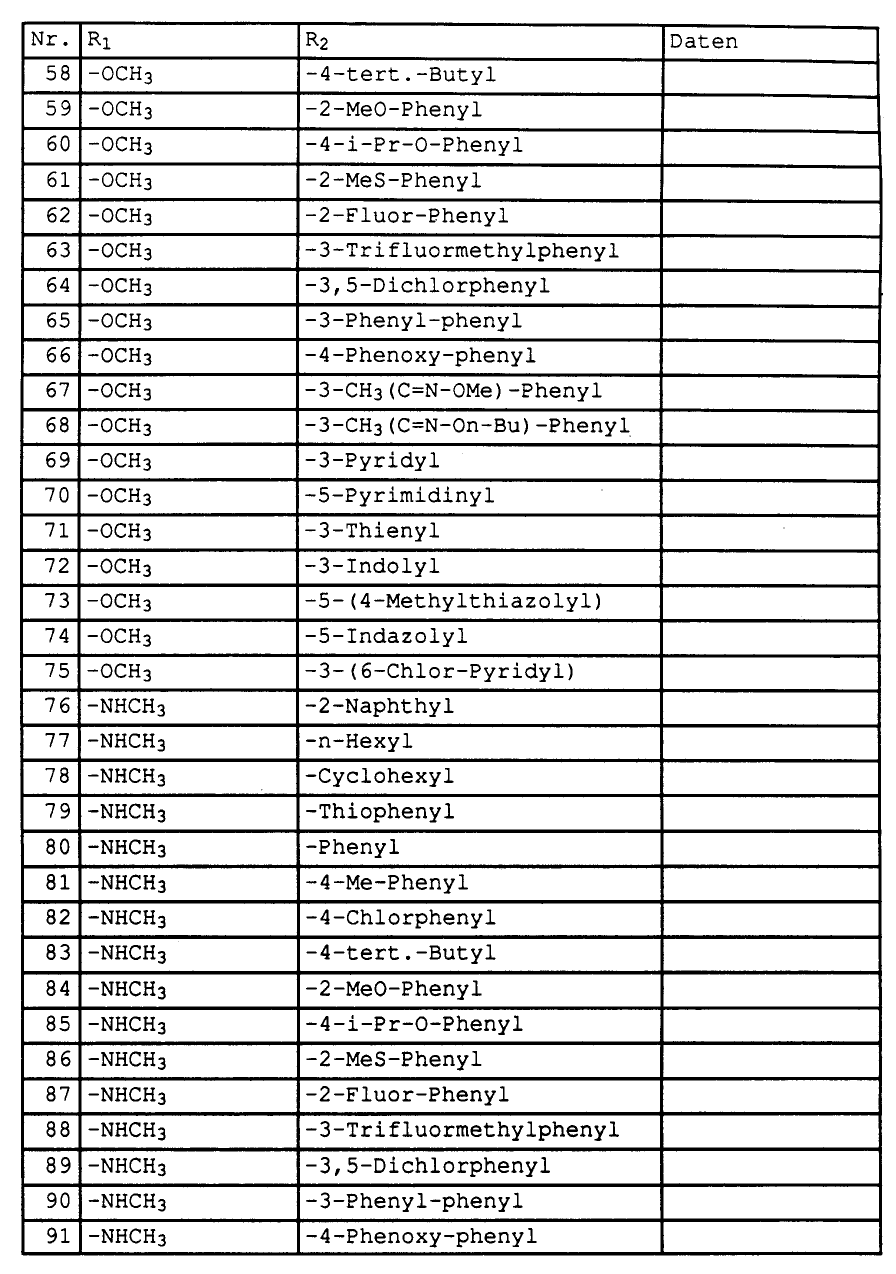

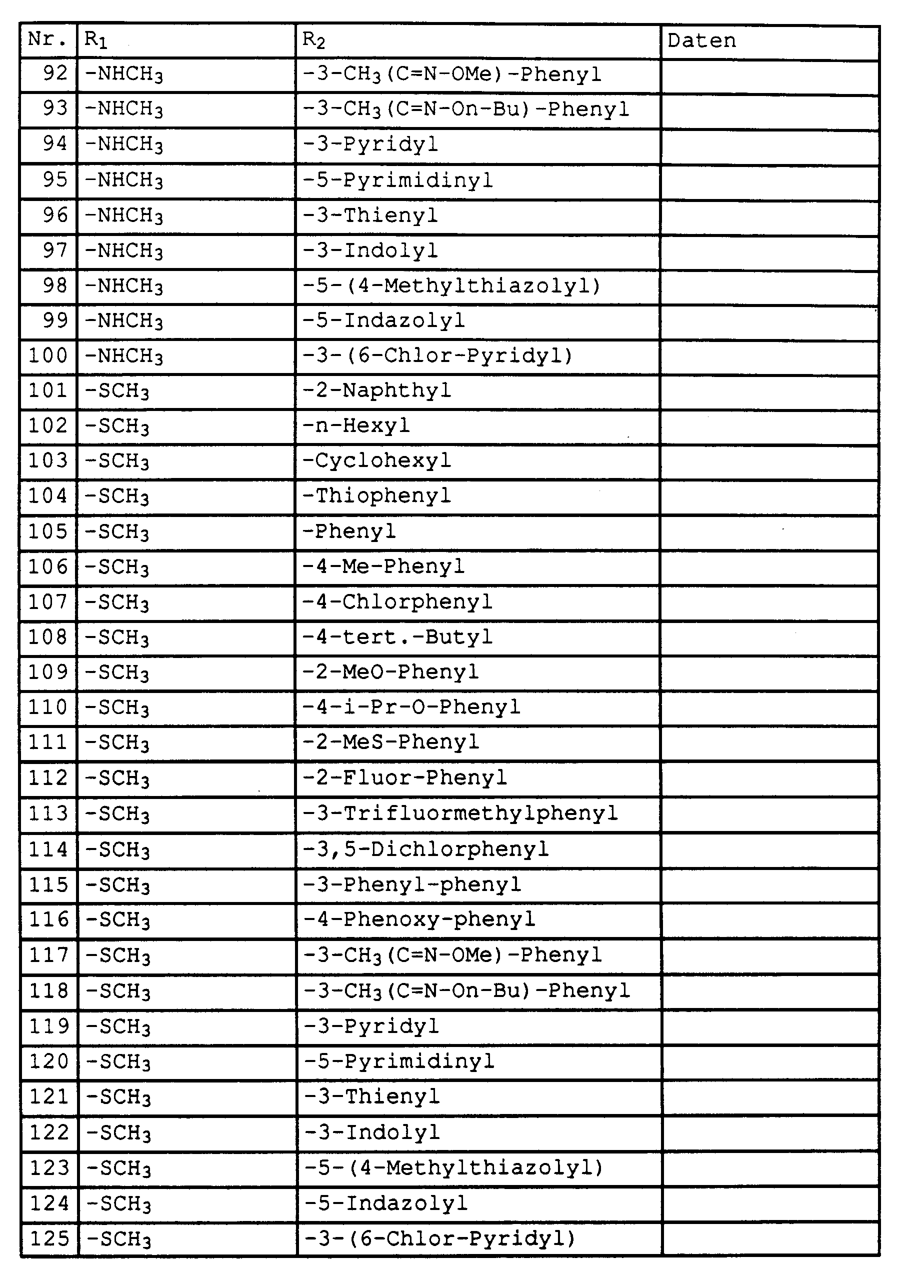

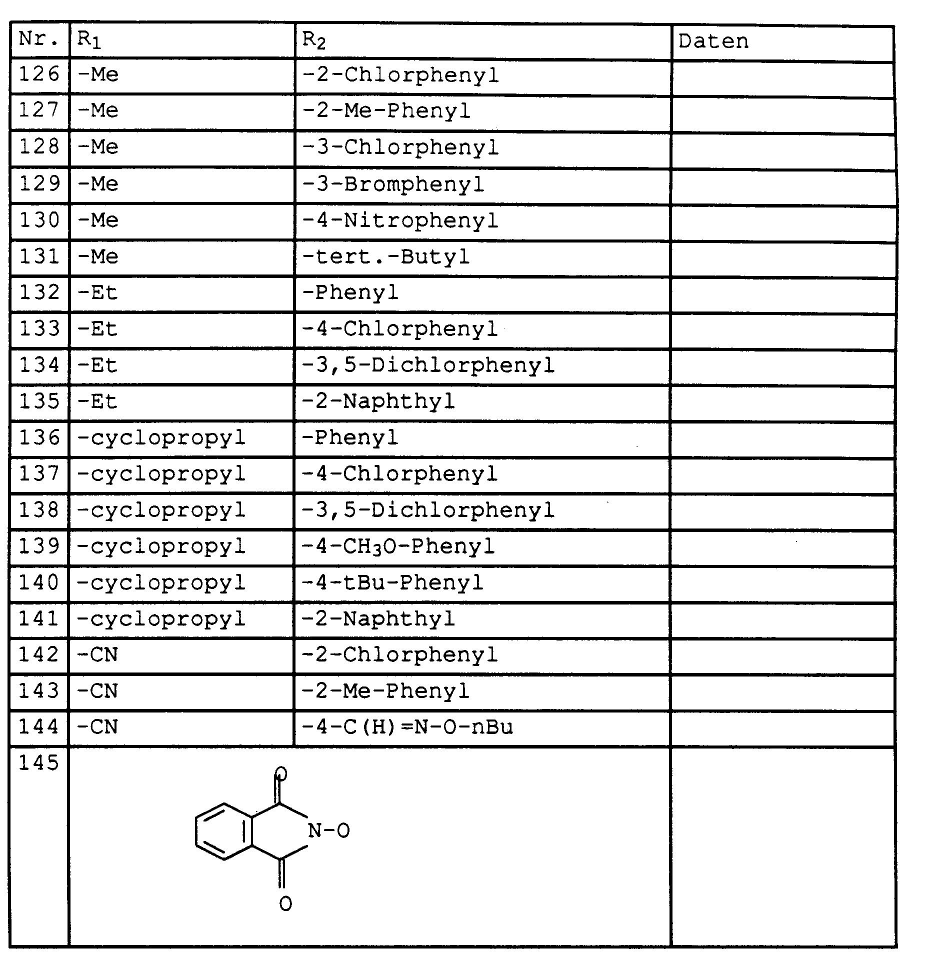

Nach den genannten Verfahren können beispielsweise die folgenden o-Iminooxymethylbenzoesäurechloride IV hergestellt werden.

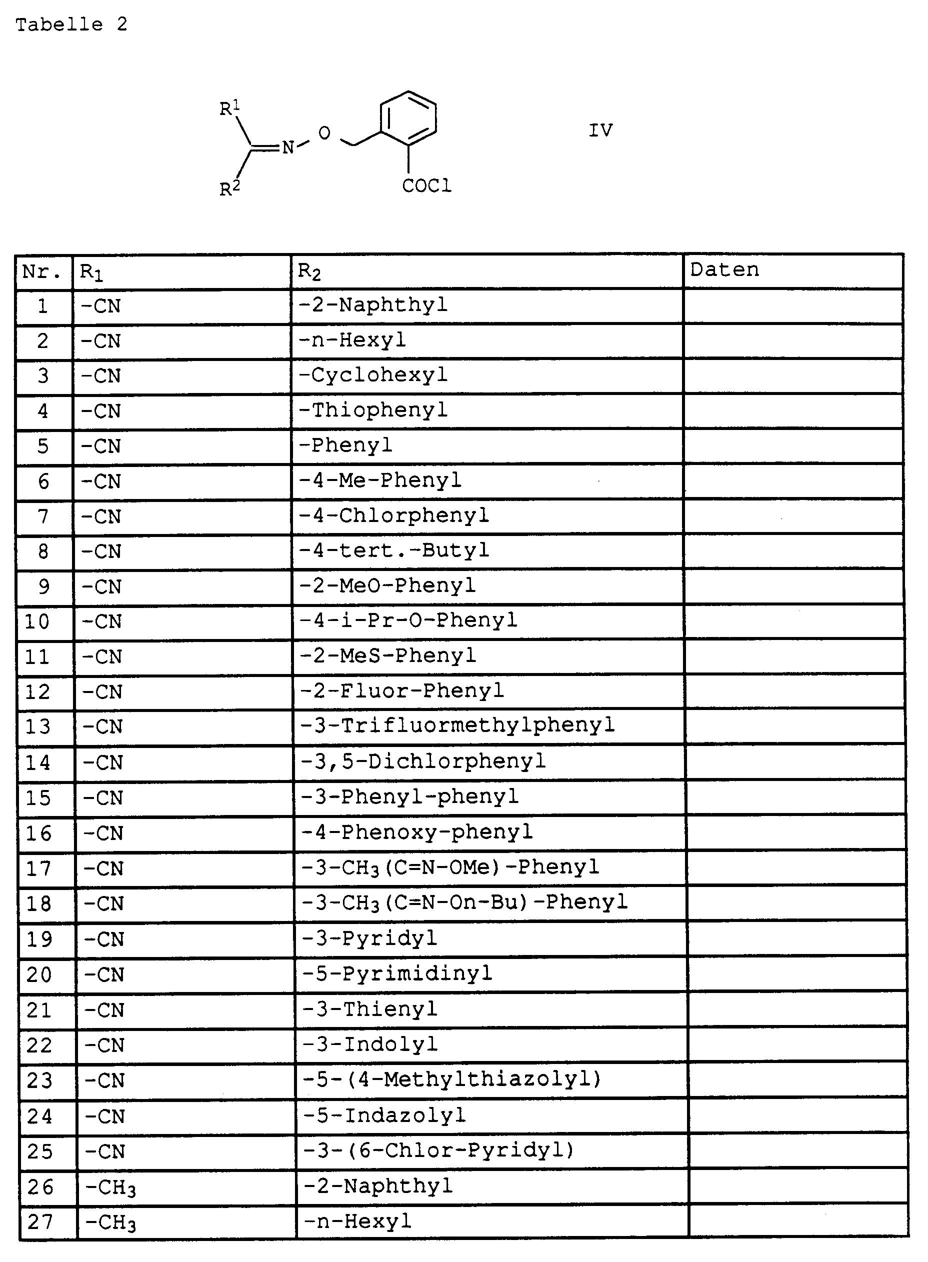

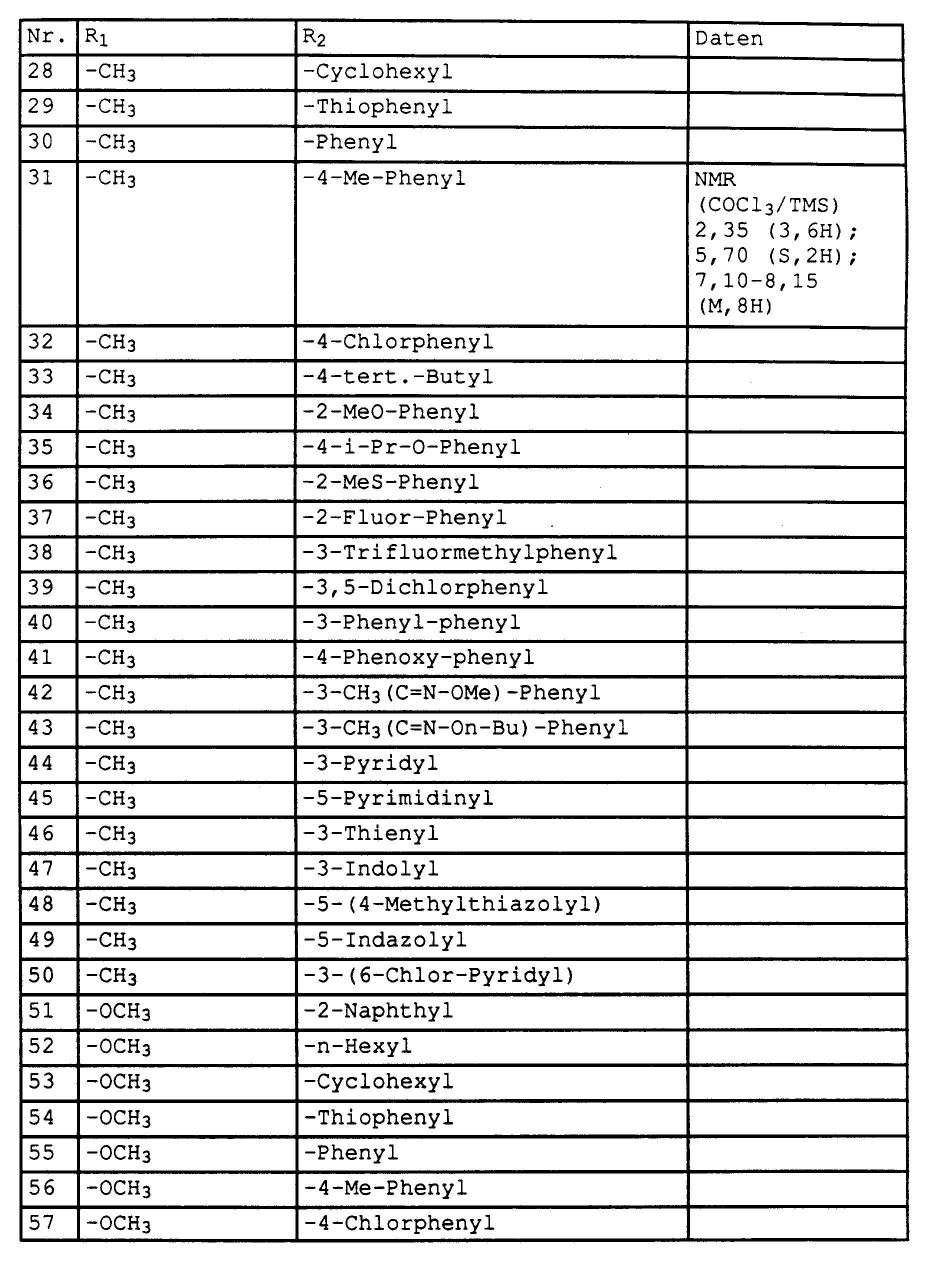

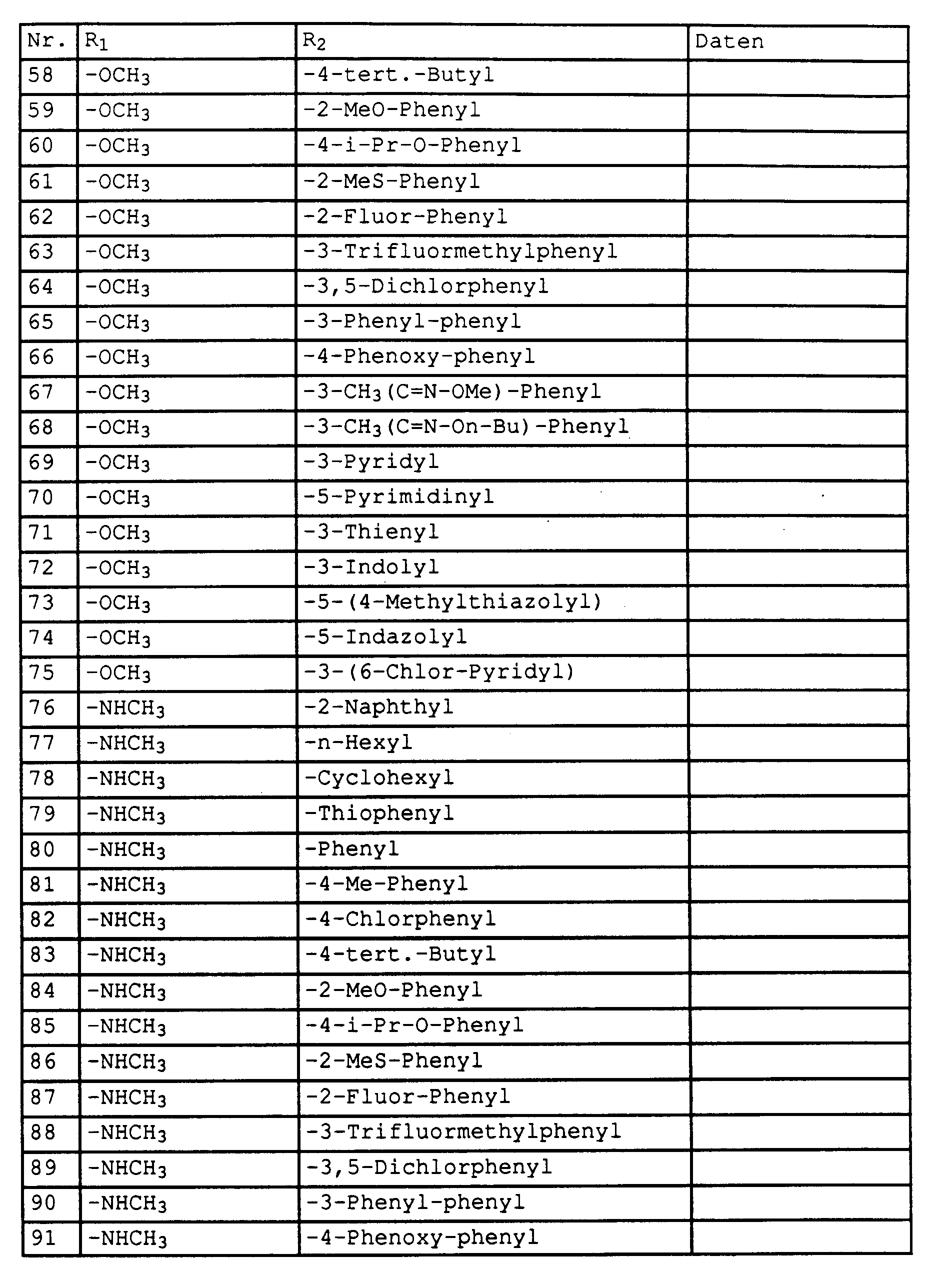

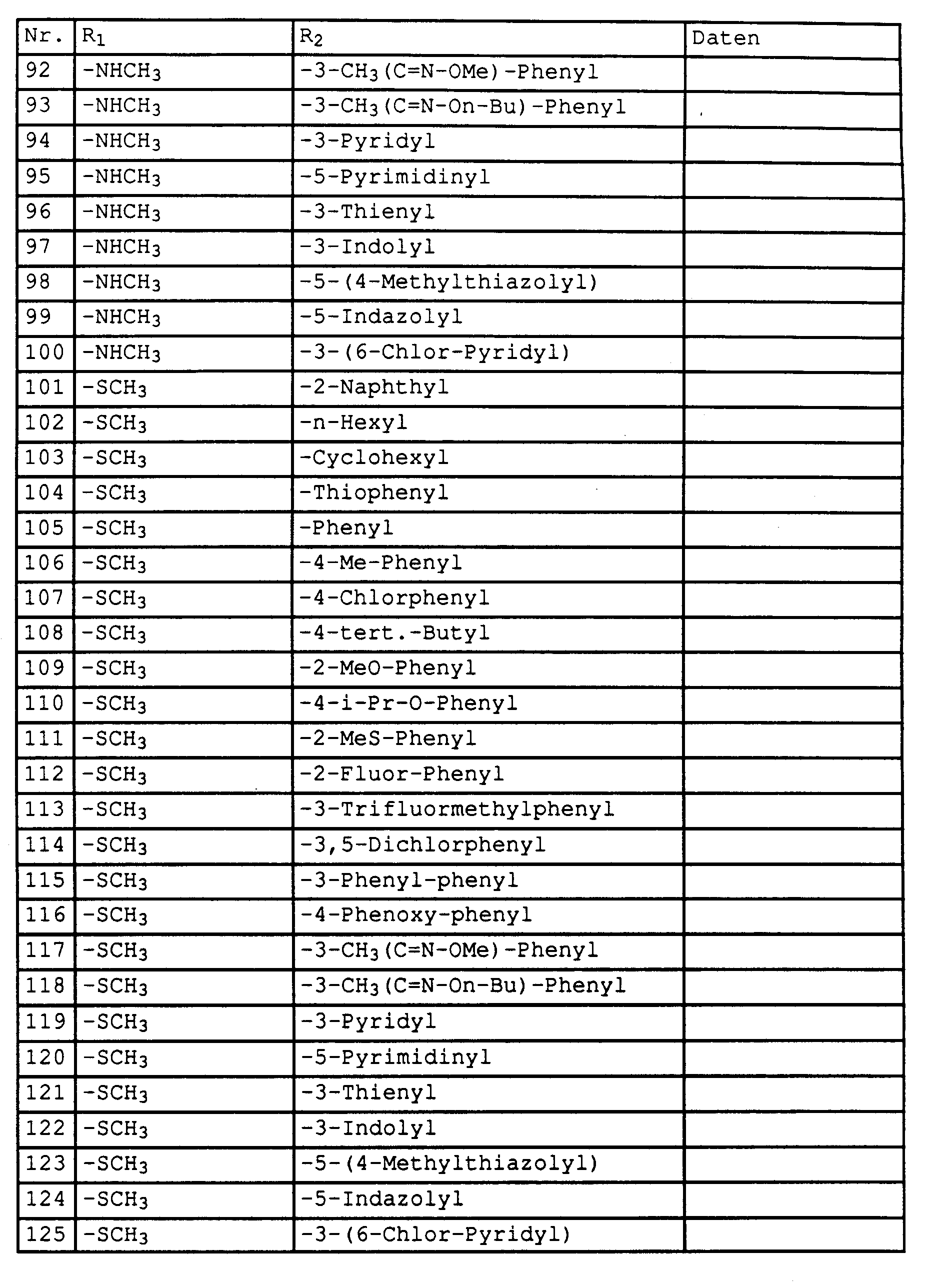

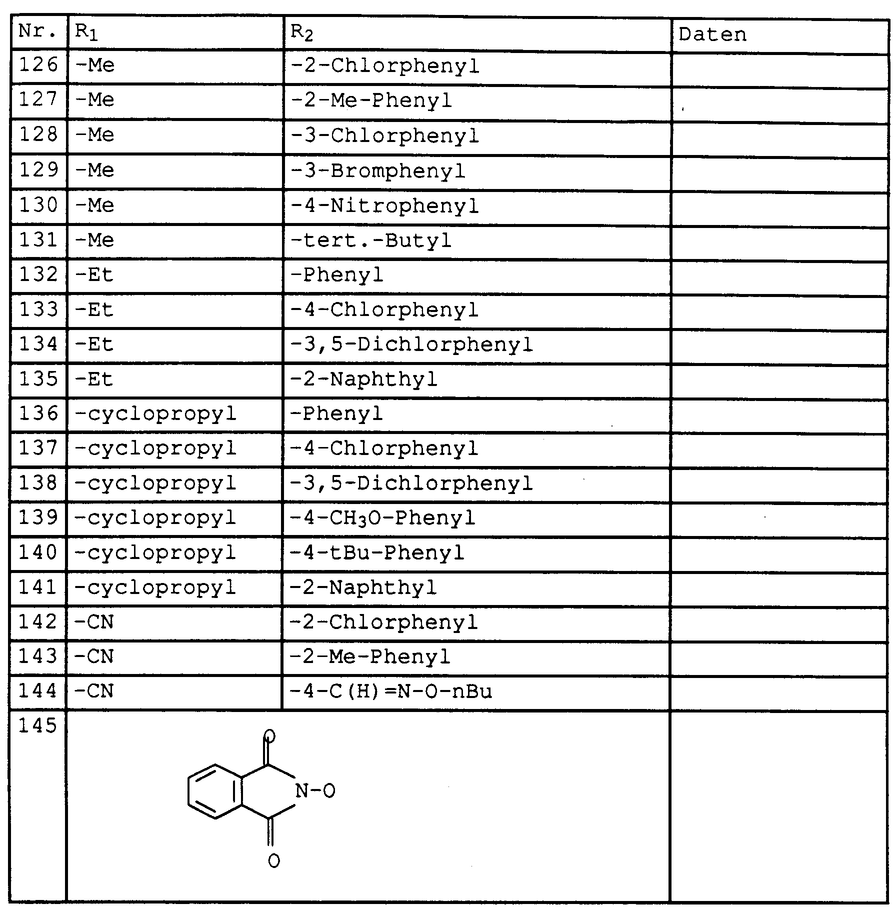

Nach den genannten Verfahren können beispielsweise die folgenden o-Iminooxymethylbenzoesäurecyanide VI hergestellt werden.

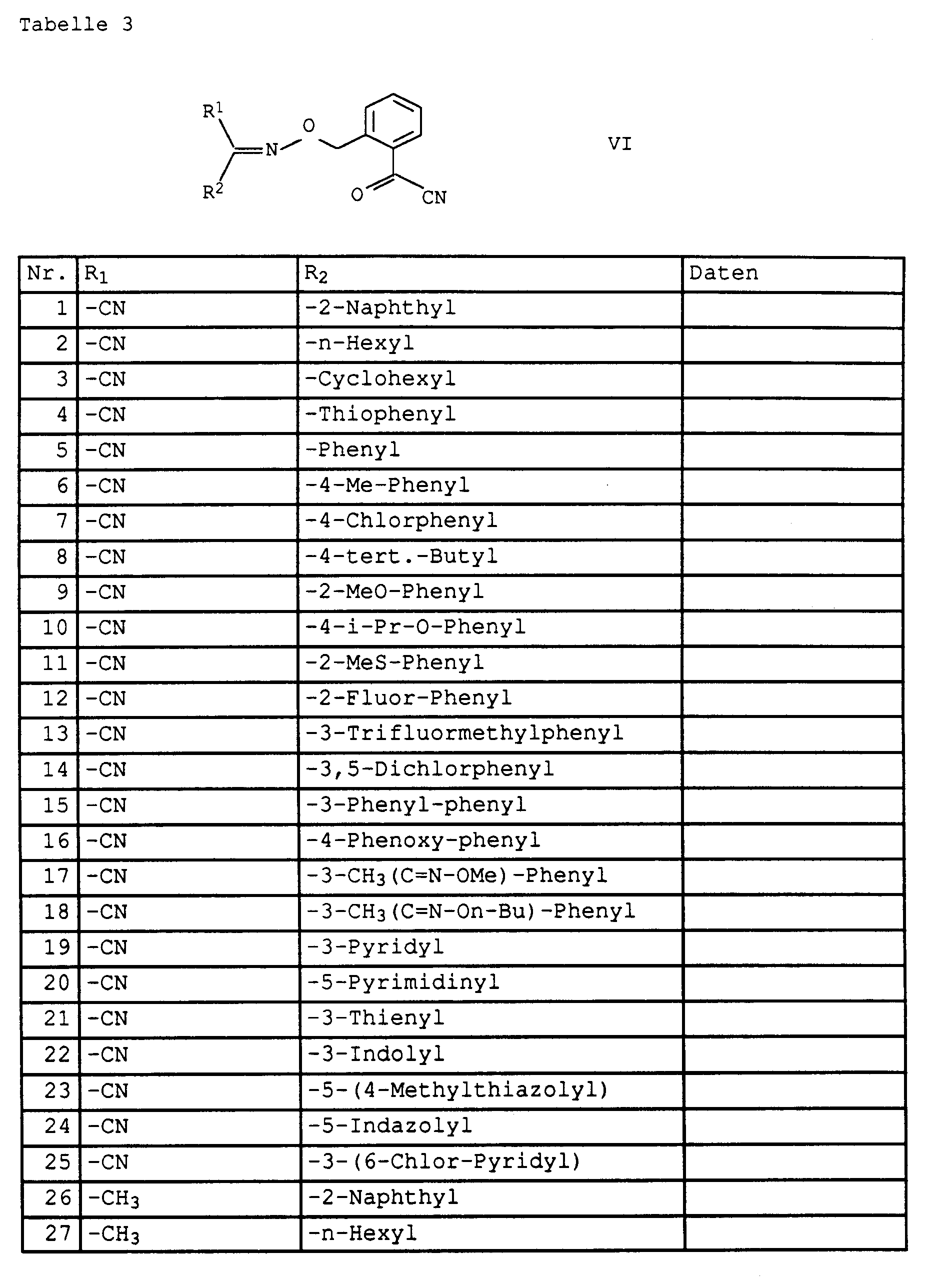

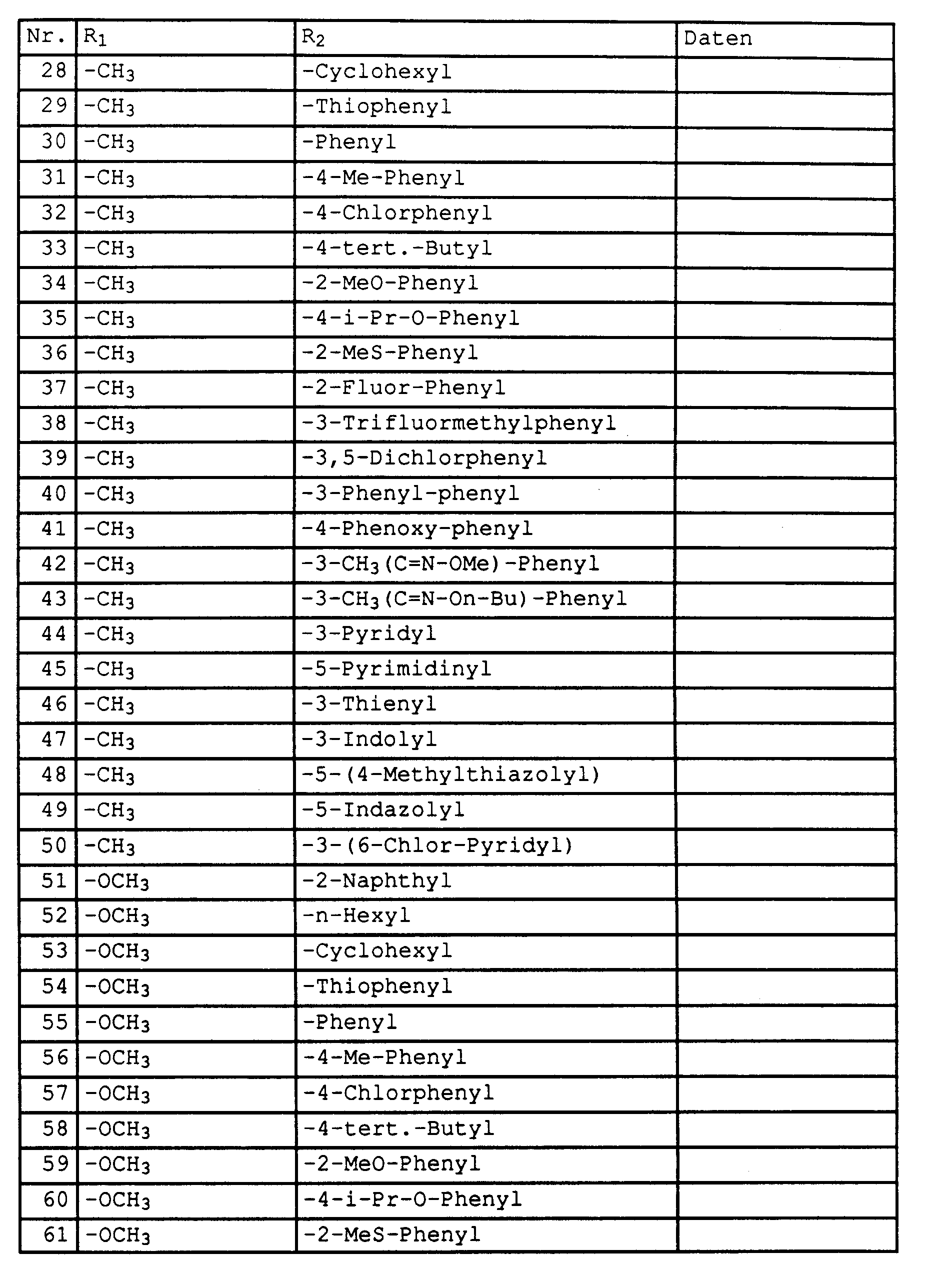

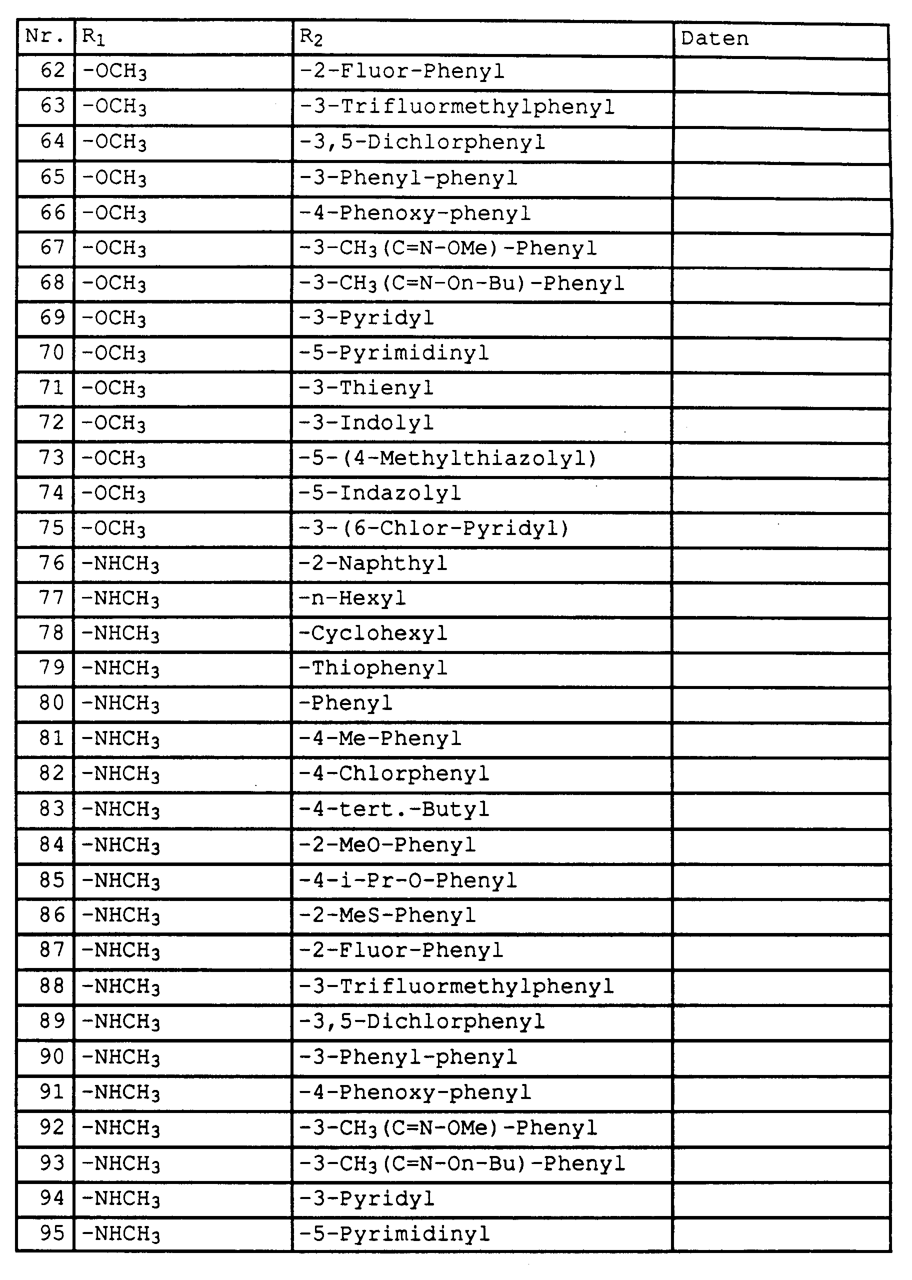

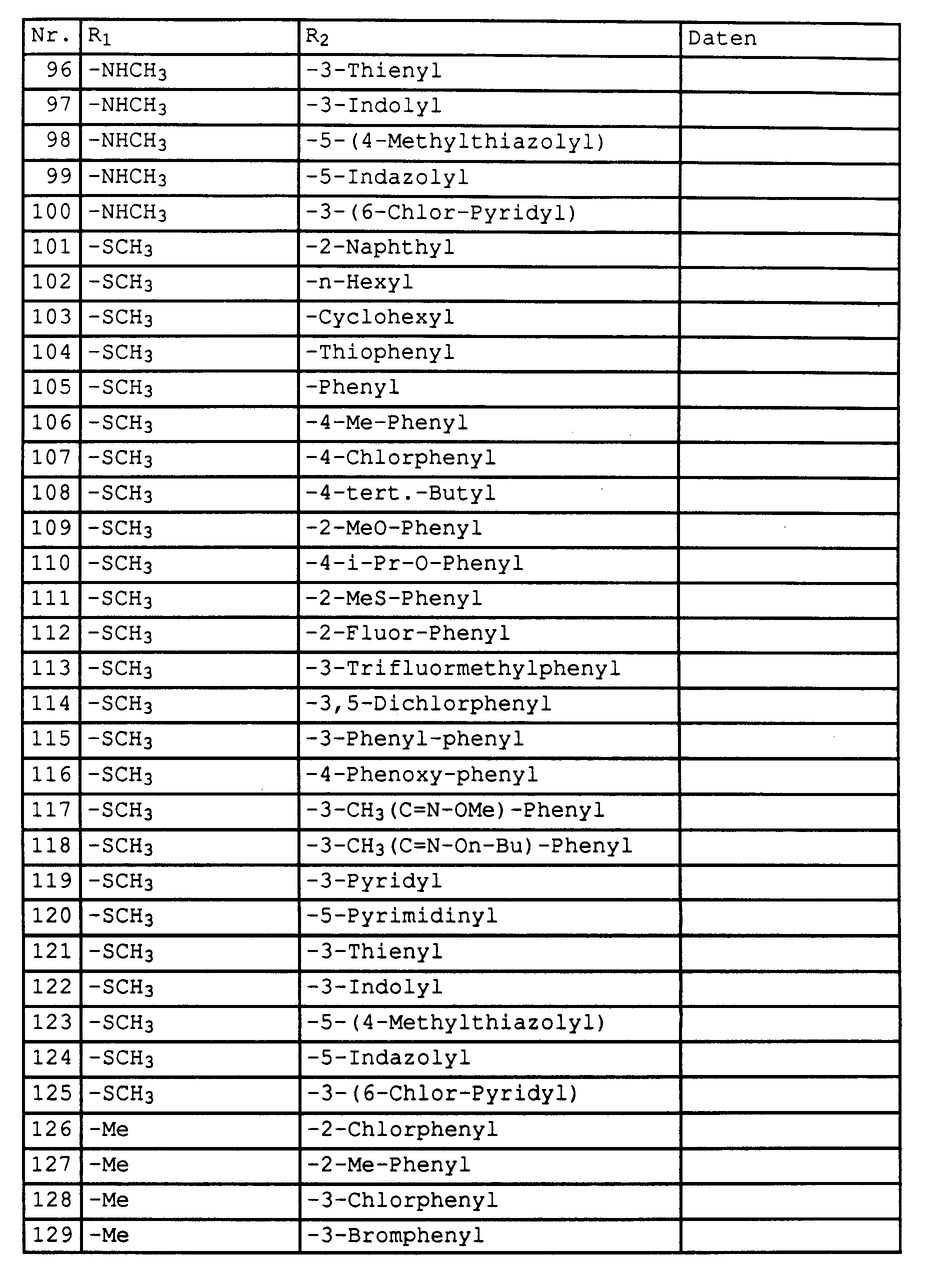

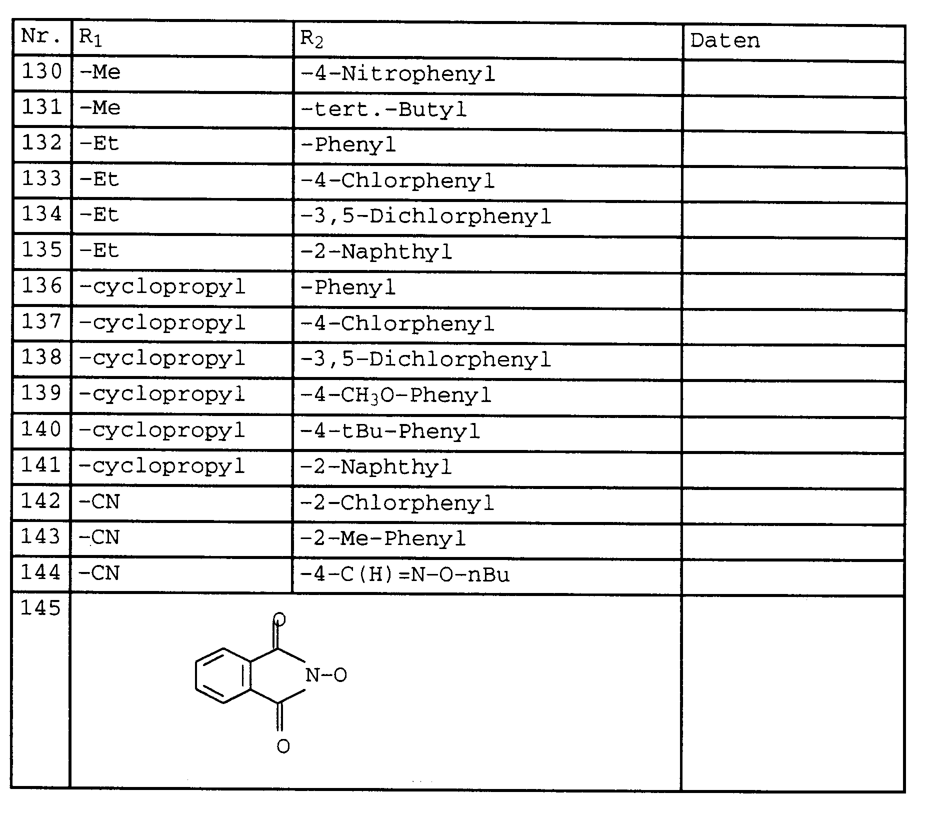

## Patentansprüche

1. Verfahren zur Herstellung von o-Iminooxymethylbenzoesäuren der allgemeinen Formel I 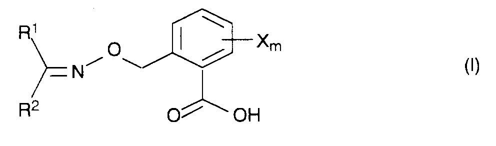 in der
m 0 oder eine ganze Zahl von 1 bis 3 bedeutet,
X ggf. verzweigtes C₁-C₄-Alkyl, ggf. verzweigtes C₁-C₄-Alkoxy, Nitro, Cyano, Halogen bedeutet,
R¹,R² gleich oder verschieden sind und Wasserstoff, Cyano, Hydroxy, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₆-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, Benzylamino, C₁-C₄-Alkylcarbonyl, ggf. subst. Phenylcarbonyl, ggf. subst. Benzylcarbonyl, C₁-C₄-Alkoxycarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Hetaryl-C₁-C₄-alkyl, ggf. subst. Hetaryl-C₂-C₄-alkenyl, ggf. subst. Hetaryloxy-C₁-C₄-alkyl, ggf. subst. Hetarylthio-C₁-C₄-alkyl, ggf. subst. Heterocyclyl, ggf. subst. Heterocyclyloxy bedeuten, oder
N(R⁶)₂ bedeuten, wobei die Bedeutungen von R⁶ gleich oder verschieden sind und Wasserstoff, C₁-C₆-Alkyl, ggf. subst. Phenyl bedeuten, oder
CO-N(R⁷)₂ bedeuten, wobei die Bedeutungen von R⁷ gleich oder verschieden sind und Wasserstoff, C₁-C₆-Alkyl, ggf. subst. Phenyl bedeuten,
wobei "ggf. subst." neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoxyimino-C₁-C₂-alkyl, Aryl, Aryloxy, Benzyloxy, Hetaryl, Hetaryloxy, C₃-C₆-Cycloalkyl, Heterocyclyl, Heterocyclyloxy bedeuten, oder
R¹ und R² zusammen mit dem C-Atom, dessen Substituenten sie sind, einen carbo- oder heterocyclischen Ring bilden können, der durch die unter "ggf. susbt." oben genannten Reste substituiert sein kann, oder
R¹ oder R² Halogen bedeutet oder die Gruppe 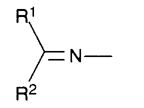 den Rest 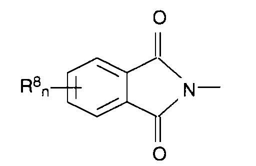 bedeutet, wobei
n die ganzen Zahlen von 1 bis 4 bedeutet, und
die Reste R⁸ gleich oder verschieden sind und Wasserstoff, Halogen, Cyano, Nitro, ggf. subst. C₁-C₄-Alkyl, C₁-C₄-Alkoxy, C₁-C₄-Halogenalkyl, C₁-C₄-Halogenalkoxy, Aryl, Aryloxy, Benzyloxy, Hetaryl, Hetaryloxy bedeuten,
dadurch gekennzeichnet, daß man ein Oxim der allgemeinen Formel II 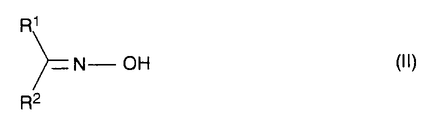 in dem R¹ und R² die oben genannte Bedeutung haben, mit einem Lacton der allgemeinen Formel III, 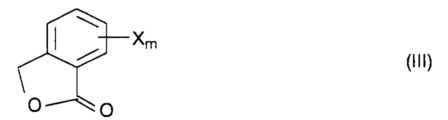 in dem X und m die oben gegebene Bedeutung haben, gegebenenfalls in Gegenwart einer Base oder eines Verdünnungsmittels oder ihrer Mischung umsetzt.

2. Verfahren zur Herstellung von o-Iminooxymethylbenzoesäuren der allgemeinen Formel I gemäß Anspruch 1, dadurch gekennzeichnet, daß man das Oxim der Formel II und das Lacton der Formel III in Gegenwart einer Base und gegebenenfalls in Gegenwart eines Verdünnungsmittels umsetzt.

3. Verfahren nach Anspruch 1, dadurch gekennzeichnet, daß man als Base ein Alkalimetallalkoholat oder ein Metallhydrid verwendet und die Reaktion in einem aprotischen, polaren Lösungsmittel durchführt.

4. Verfahren zur Herstellung von o-Iminooxymethylbenzoesäuren der allgemeinen Formel I gemäß Anspruch 1, dadurch gekennzeichnet, daß man
a) ein Oxim der allgemeinen Formel II, 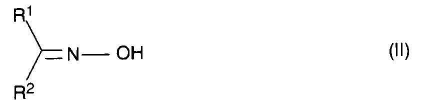 in dem R¹ und R² die oben genannte Bedeutung haben, in Gegenwart eines Verdünnungsmittels mittels einer Base in das entsprechende Salz überführt,
b) dieses mit einem Lacton der allgemeinen Formel III, 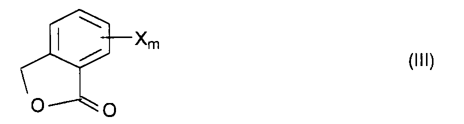 in dem X und m die oben genannte Bedeutung haben, mischt,
c) das Gemisch in Lösung in einem Temperaturbereich zwischen 20°C und 250°C weiter umsetzt oder
d) das Verdünnungsmittel abdestilliert und das Gemisch in einem Temperaturbereich zwischen 50°C und 250°C in der Schmelze umsetzt.

5. Verfahren nach Anspruch 4, dadurch gekennzeichnet, daß man den Verfahrensschritt (a) bei Temperaturen zwischen 0°C und 100°C vornimmt und ein Alkalimetallalkoholat oder ein Metallhydrid als Base verwendet.

6. Verbindungen der Formel I gemäß Anspruch 1.

7. Verwendung der Verbindungen I gemäß Anspruch 1 als Zwischenprodukte.

8. Verwendung der Verbindungen I gemäß Anspruch 1 zur Herstellung von o-Iminooxymethylbenzoesäure-chloriden der allgemeinen Formel IV, 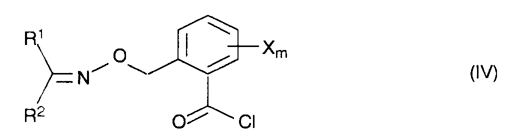 in der der Index und die Substituenten die in Anspruch 1 gegebene Bedeutung haben.

9. Verwendung der Verbindungen I gemäß Anspruch 1 zur Herstellung von o-Iminooxymethylbenzoesäure-cyaniden der allgemeinen Formel VI, 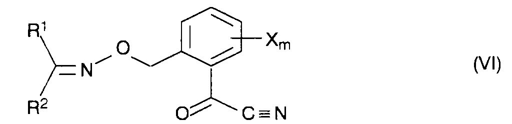 in der der Index und die Substituenten die in Anspruch 1 gegebene Bedeutung haben.

10. o-Iminooxymethylbenzoesäure-chloride der allgemeinen Formel IV, 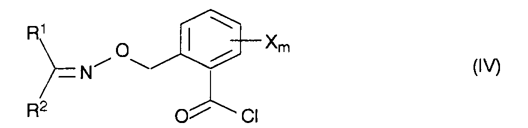 in der der Index und die Substituenten die in Anspruch 1 gegebene Bedeutung haben.

11. o-Iminooxymethylbenzoesäure-cyanide der allgemeinen Formel VI, 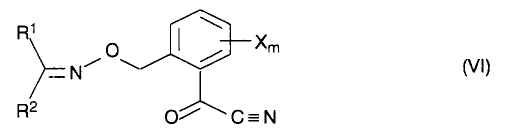 in der der Index und die Substituenten die in Anspruch 1 gegebene Bedeutung haben.

## Claims

1. A process for preparing an o-iminooxymethylbenzoic acid of the general formula I 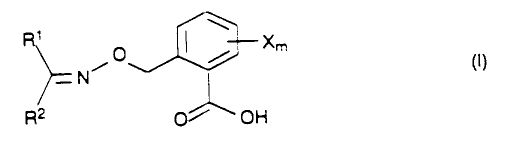 where
m is 0 or an integer from 1 to 3,
X is unbranched or branched C₁-C₄-alkyl, unbranched or branched C₁-C₄-alkoxy, nitro, cyano or halogen,
R¹ and R² are identical or different and each is hydrogen, cyano, hydroxyl, unbranched or branched C₁-C₁₀-alkyl, C₁-C₄-haloalkyl, C₃-C₆-cycloalkyl, C₃-C₆-halocyclcalkyl, C₃-C₆-cyclcalkyl-C₁-C₄-alkyl, C₁-C₄-alkoxy-C₁-C₄-alkyl, C₁-C₄-alkylthio-C₁-C₄-alkyl, arylthio-C₁-C₄-alkyl, C₂-C₆-alkenyl, C₂-C₆-haloalkenyl, C₃-C₆-cyclcalkenyl, C₃-C₆-halocycloalkenyl, C₂-C₆-alkynyl, C₁-C₆-alkoxy, C₁-C₆-haloalkoxy, C₁-C₄-alkylthio, benzylthio, benzylamino, C₁-C₄-alkylcarbonyl, unsubstituted or substituted phenylcarbonyl, unsubstituted or substituted benzylcarbonyl, C₁-C₄-alkoxycarbonyl, unsubstituted or substituted phenoxycarbonyl, unsubstituted or substituted benzyloxycarbonyl, unsubstituted or substituted aryl, unsubstituted or substituted aryloxy, unsubstituted or substituted arylthio, unsubstituted or substituted arylC₁-C₄-alkyl, unsubstituted or substituted aryl-C₂-C₄-alkenyl, unsubstituted or substituted aryloxy-C₁-C₄-alkyl, unsubstituted or substituted arylthio-C₁-C₄-alkyl, unsubstituted or substituted hetaryl, unsubstituted or substituted hetaryloxy, unsubstituted or substituted hetarylthio, unsubstituted or substituted hetaryl-C₁-C₄-alkyl, unsubstituted or substituted hetaryl-C₂-C₄-alkenyl, unsubstituted or substituted hetaryloxy-C₁-C₄-alkyl, unsubstituted or substituted hetarylthio-C₁-C₄-alkyl, unsubstituted or substituted heterocyclyl, unsubstituted or substituted heterocyclyloxy, or
N(R⁶)₂, where the meanings of the two R⁶ radicals are identical or different and are hydrogen, C₁-C₆-alkyl or unsubstituted or substituted phenyl, or
CO-N(R⁷)₂, where the meanings of the two R⁷ radicals are identical or different and are hydrogen, C₁-C₆-alkyl or unsubstituted or substituted phenyl,
where unsubstituted or substituted includes, in addition to hydrogen, the radicals halogen, cyano, nitro, C₁-C₄-alkyl, C₁-C₄-alkoxy, C₁-C₄-haloalkyl, C₁-C₄-haloalkoxy, C₁-C₁₀-alkoximino-C₁-C₂-alkyl, aryl, aryloxy, benzyloxy, hetaryl, hetaryloxy, C₃-C₆-cycloalkyl, heterocyclyl or heterocyclyloxy, or
R¹ and R², together with the C atom whose substituents they are, form a carbocyclic or heterocyclic ring which can be substituted by the radicals mentioned above under unsubstituted or substituted, or
R¹ or R² is halogen, or the group 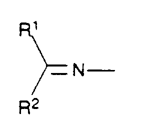 is the radical 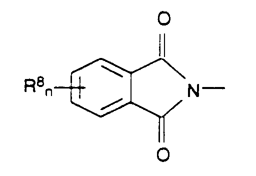 where
n is an integer from 1 to 4, and
the radicals R⁸ are identical or different and each is hydrogen, halogen, cyano, nitro, unsubstituted or substituted C₁-C₄-alkyl, C₁-C₄-alkoxy, C₁-C₄-haloalkyl, C₁-C₄-haloalkoxy, aryl, aryloxy, benzyloxy, hetaryl or hetaryloxy,
which comprises reacting an oxime of the general formula II 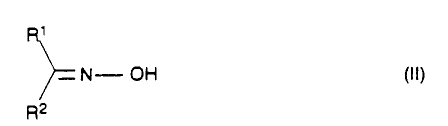 where R¹ and R² have the abovementioned meanings, with a lactone of the general formula III 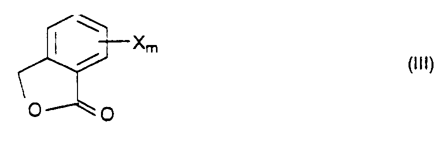 where X and m have the abovementioned meanings, if appropriate in the presence of a base or of a diluent or a mixture thereof.

2. A process for preparing an o-iminooxymethylbenzoic acid of the general formula I as claimed in claim 1, wherein the oxime of the formula II and the lactone of the formula III are reacted in the presence of a base and if appropriate in the presence of a diluent.

3. A process as claimed in claim 1, wherein the base used is an alkali metal alkoxide or a metal hydride and the reaction is carried out in an aprotic polar solvent.

4. A process for preparing an o-iminooxymethylbenzoic acid of the general formula I as claimed in claim 1, wherein
a) an oxime of the general formula II 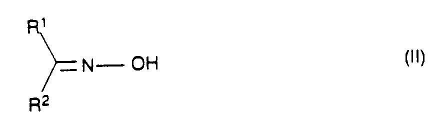 in which R¹ and R² have the abovementioned meanings, is converted into the corresponding salt by means of a base in the presence of a diluent,
b) this salt is mixed with a lactone of the general formula III 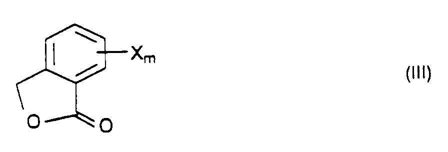 where X and m have the abovementioned meanings,
c) the mixture is further reacted in solution at from 20 to 250°C, or
d) the diluent is distilled off and the mixture is reacted in the molten state at from 50 to 250°C.

5. A process as claimed in claim 4, wherein process step (a) is carried out at from 0 to 100°C and an alkali metal alkoxide or a metal hydride is used as the base.

6. A compound of the formula I as claimed in claim 1.

7. The use of a compound I as claimed in claim 1 as an intermediate.

8. The use of a compound I as claimed in claim 1 for preparing an o-iminooxymethylbenzoyl chloride of the general formula IV 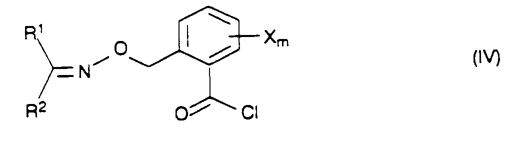 where the index and the substituents have the meanings given in claim 1.

9. The use of a compound I as claimed in claim 1 for preparing an o-iminooxymethylbenzoyl cyanide of the general formula VI 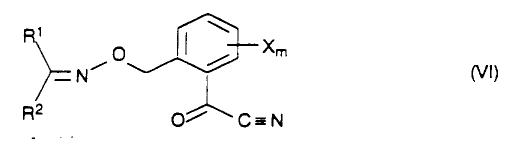 where the index and the substituents have the meanings given in claim 1.

10. An o-iminooxymethylbenzoyl chloride of the general formula IV 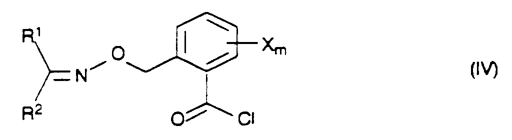 where the index and the substituents have the meanings given in claim 1.

11. An o-iminooxymethylbenzoyl cyanide of the general formula VI 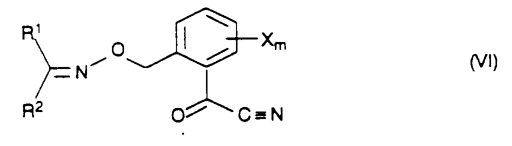 where the index and the substituents have the meanings given in claim 1.

## Revendications

1. Procédé de préparation d'acides o-iminooxyméthylbenzoïques de formule générale I 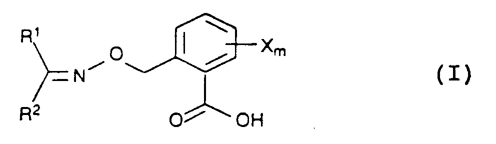 dans laquelle
m est égal à O ou à un nombre entier allant de 1 à 3,
X représente un groupe alkyle en C1-C4 éventuellement ramifié, alcoxy en C1-C4 éventuellement ramifié, nitro, cyano, un halogène,
R¹ et R², ayant des significations identiques ou différentes, représentent chacun l'hydrogène, un groupe cyano, hydroxy, alkyle en C1-C10 éventuellement ramifié, halogénoalkyle en C1-C4, cycloalkyle en C3-C6, halogénocycloalkyle en C3-C6, (cycloalkyle en C3-C6)alkyle en C1-C6, (alcoxy en C1-C4)alkyle en C1-C4, (alkylthio en C1-C4)alkyle en C1-C4, arylthio-alkyle en C1-C4, alcényle en C2-C6, halogénoalcényle en C2-C6, cycloalcényle en C3-C6, halogénocycloalcényle en C3-C6, alcynyle en C2-C6, alcoxy en C1-C6, halogénoalcoxy en C1-C6, alkylthio en C1-C4, benzylthio, benzylamino, (alkyle en C1-C4)-carbonyle, phénylcarbonyle éventuellement substitué, benzylcarbonyle éventuellement substitué, (alcoxy en C1-C4)carbonyle, phénoxycarbonyle éventuellement substitué, benzyloxycarbonyle éventuellement substitué, aryle éventuellement substitué, aryloxy éventuellement substitué, arylthio éventuellement substitué, aryl-alkyle en C1-C4 éventuellement substitué, aryl-alcényle en C2-C4 éventuellement substitué, aryloxy-alkyle en C1-C4 éventuellement substitué, arylthio-alkyle en C1-C4 éventuellement substitué, hétéroaryle éventuellement substitué, hétéroaryloxy éventuellement substitué, hétéroarylthio éventuellement substitué, hétéroaryl-alkyle en C1-C4 éventuellement substitué, hétéroaryl-alcényle en C2-C4 éventuellement substitué, hétéroaryloxy-alkyle en C1-C4 éventuellement substitué, hétéroarylthio-alkyle en C1-C4 éventuellement substitué, hétérocyclyle éventuellement substitué, hétérocyclyloxy éventuellement substitué, ou bien
N(R⁶)₂, les deux symboles R⁶ ayant des significations identiques ou différentes et représentant chacun l'hydrogène, un groupe alkyle en C1-C6, un groupe phényle éventuellement substitué, ou bien
CO-N(R⁷)₂, les deux symboles R⁷ ayant des significations identiques ou différentes et représentant chacun l'hydrogène, un groupe alkyle en C1-C6, un groupe phényle éventuellement substitué,
l'expression "éventuellement substitué" indiquant qu'outre l'hydrogène, on peut trouver des substituants halogéno, cyano, nitro, alkyle en C1-C4, alcoxy en C1-C4, halogénoalkyle en C1-C4, halogénoalcoxy en C1-C4, (alcoxy en C1-C10)iminoalkyle en C1-C2, aryle, aryloxy, benzyloxy, hétéroaryle, hétéroaryloxy, cycloalkyle en C3-C6, hétérocyclyle, hétérocyclyloxy, ou bien
R¹ et R² forment ensemble et avec l'atome de carbone dont ils sont substituants un noyau carboxycyclique ou hétérocyclique qui peut porter les substituants dont il a été question ci-dessus en référence à l'expression "éventuellement substitué", ou bien R¹ ou R² représente un halogène, ou bien le groupe 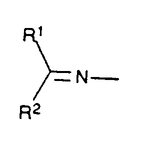 consiste en un groupe 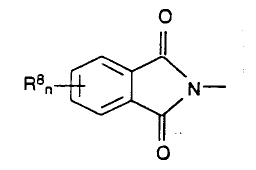 dans lequel
n est un nombre entier allant de 1 à 4 et
les symboles R⁸, ayant des significations identiques ou différentes, représentent chacun l'hydrogène, un halogène, un groupe cyano, nitro, alkyle en C1-C4 éventuellement substitué, alcoxy en C1-C4, halogénoalkyle en C1-C4, halogénoalcoxy en C1-C4, aryle, aryloxy, benzyloxy, hétéroaryle, hétéroaryloxy,
caractérisé par le fait que l'on fait réagir une oxime de formule générale II 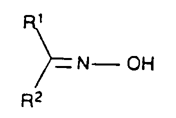 dans laquelle R¹ et R² ont les significations indiquées ci-dessus, avec une lactone de formule générale III, 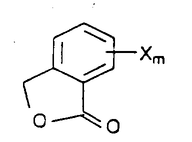 dans laquelle X et n ont les signification indiquées ci-dessus, le cas échéant en présence d'une base ou d'un diluant ou de leur mélange.

2. Procédé de préparation des acides o-iminooxyméthylbenzoïques de formule générale I selon la revendication 1, caractérisé par le fait qu'on fait réagir l'oxime de formule II et la lactone de formule III en présence d'une base et le cas échéant en présence d'un diluant.

3. Procédé selon la revendication 1, caractérisé par le fait que la base utilisée est un alcoolate de métal alcalin ou un hydrure métallique et par le fait que la réaction est effectuée dans un solvant polaire aprotonique.

4. Procédé de préparation des acides o-iminooxyméthylbenzoïques de formule générale I selon la revendication 1, caractérisé par le fait que
**a)** on convertit une oxime de formule générale II 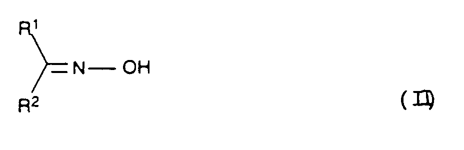 dans laquelle R¹ et R² ont les significations indiquées ci-dessus, en présence d'un diluant, à l'aide d'une base, en le sel correspondant,
**b)** on mélange ce sel avec une lactone de formule générale III 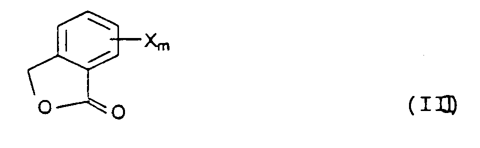 dans laquelle X et m ont les significations indiquées ci-dessus,
**c)** on fait réagir le mélange en solution dans un intervalle de température de 20 à 250° C, ou bien
**d)** on distille le diluant et on fait réagir le mélange à l'état fondu dans l'intervalle de température de 50 à 250° C.

5. Procédé selon la revendication 4, caractérisé par le fait que, au stade opératoire a), on observe des températures de 0 à 100° C et on utilise en tant que base un alcoolate de métal alcalin ou un hydrure métallique.

6. Les composés de formule I selon la revendication 1.

7. Utilisation des composés I selon la revendication 1 en tant que produits intermédiaires.

8. Utilisation des composés I selon la revendication 1 pour la préparation de chlorures d'acides o-iminooxyméthylbenzoïques de formule générale IV 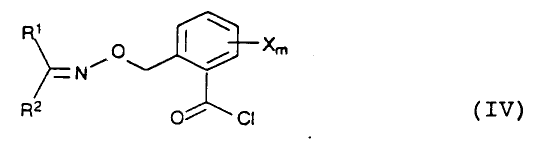 dans laquelle l'indice et les symboles ont les significations indiquées dans la revendication 1.

9. Utilisation des composés I selon la revendication 1 pour la préparation des cyanures d'acides o-iminooxyméthylbenzoïques de formule générale VI 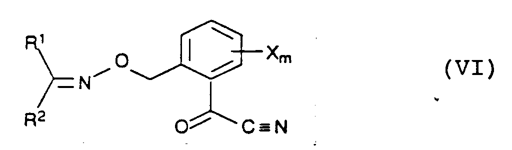 dans laquelle l'indice et les symboles ont les significations indiquées dans la revendication 1.

10. Les chlorures d'acides o-iminooxyméthylbenzoïques de formule générale IV 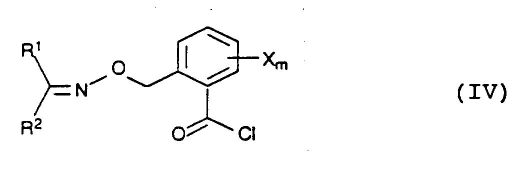 dans laquelle l'indice et les symboles ont les significations indiquées dans la revendication 1.

11. Les cyanures d'acides o-iminooxyméthylbenzoïques de formule générale VI 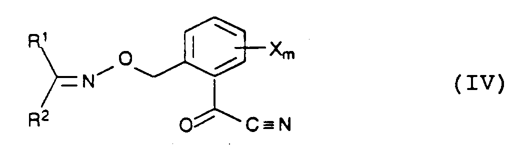 dans laquelle l'indice et les symboles ont les significations indiquées dans la revendication 1.
